(19) Europäisches Patentamt
European Patent Office
Office européen des brevets

(11) EP 1 411 120 B1

(12) EUROPEAN PATENT SPECIFICATION

(45) Date of publication and mention of the grant of the patent:
**13.01.2010 Bulletin 2010/02**

(21) Application number: **02741410.1**

(22) Date of filing: **03.07.2002**

(51) Int Cl.:
***C12Q 1/68*** *(2006.01)*

(86) International application number:
**PCT/JP2002/006754**

(87) International publication number:
**WO 2003/004640 (16.01.2003 Gazette 2003/03)**

# (54) DNA ARRAYS FOR MEASURING SENSITIVITY TO ANTICANCER AGENT

DNA-ARRAYS ZUR MESSUNG DER EMPFINDLICHKEIT GEGENÜBER EINEM ANTIKREBSMITTEL

RESEAUX D'ADN PERMETTANT DE MESURER LA SENSIBILITE A UN AGENT ANTICANCEREUX

(84) Designated Contracting States:
**AT BE BG CH CY CZ DE DK EE ES FI FR GB GR IE IT LI LU MC NL PT SE SK TR**

(30) Priority: **05.07.2001 JP 2001204448**
**07.08.2001 JP 2001239181**

(43) Date of publication of application:
**21.04.2004 Bulletin 2004/17**

(73) Proprietor: **TAIHO PHARMACEUTICAL COMPANY LIMITED**
**Chiyoda-ku,**
**Tokyo 101-8444 (JP)**

(72) Inventors:
- **TAKECHI, Teiji**
**Tokorozawa-shi, Saitama 359-1115 (JP)**
- **KOIZUMI, Katsuhisa**
**Hanno-shi, Saitama 357-0038 (JP)**
- **AZUMA, Atsushi**
**Tokorozawa-shi, Saitama 359-1141 (JP)**
- **FUKUSHIMA, Masakazu**
**Hanno-shi, Saitama 357-0032 (JP)**

(74) Representative: **Hartz, Nikolai**
**Wächtershäuser & Hartz**
**Patentanwälte**
**Weinstrasse 8**
**80333 München (DE)**

(56) References cited:
**WO-A-01/32928**
**WO-A1-01/75160**
**WO-A2-01/32928**
**US-A- 6 025 194**

- **SCHERF U. ET AL.: 'A gene expression database for the molecular pharmacology of cancer' NAT. GENET. vol. 24, no. 3, 2000, pages 236 - 244, XP002225993**
- **TSUNODA T. ET AL.: 'Diagnosis system of drug sensitivity of cancer using cDNA microarray and multivariate statistical anaylsis' FRONTIERS SCIENCE SERIES vol. 30, 2000, pages 16 - 17, XP002960710**
- **GREM J.L. ET AL.: 'Thymidine kinase, thymidylate synthase and dihydropyrimidine dehydrogenase profiles of cell lines of the National Cancer Institute's Anticancer Frug Scteen' CLIN. CANCER RES. vol. 7, no. 4, April 2001, pages 999 - 1009, XP002960711**
- **ISHIKAWA Y. ET AL.: 'Dihydropyrimidine dehydrogenase activity and messenger RNA level may be related to the antitumor effect of 5-fluorouracil on human tumor xenografts in nude mice' CLIN. CANCER RES. vol. 5, no. 4, 1999, pages 883 - 889, XP002960712**
- **SALONGA D. ET AL.: 'Colorectal tumors responding to 5-fluorouracil have low gene expression levels of dihydropyrimidine dehydrogenase, thymidylate synthase and thymidine phosphorylase' CLIN. CANCER RES. vol. 6, no. 4, 2000, pages 1322 - 1327, XP002960713**
- **TAKUMI OCHIAI ET AL.: 'Daicho-gan ni okeru orotate phosphoribosyl transferase (OPRT), thymidine kinase (TS), dihydropyrimidine dehydrogenase (DPD) no hatsugen to CD-DST-ho (collagen gel droplet embedded culture drug sensitivity test) ni yoru 5-FU kanjusei shiken tono kanren ...' GAN TO KAGAKU RYOHO vol. 28, no. 5, May 2001, pages 661 - 667, XP002960714**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- TAKECHI T. ET AL.: 'Screening of differentially expressed genes in 5-fluorouracil-resistant human gastrointestinal tumor cells' JPN. J. CANCER RES. vol. 92, no. 6, June 2001, pages 696 - 703, XP002960715
- ISHIKAWA Y. ET AL.: 'Dihydropyrimidine dehydrogenase and messenger RNA levels in gastric cancer: possible predictor for sensitivity to 5-fluorouracil' JPN. J. CANCER RES. vol. 91, no. 1, 2000, pages 105 - 112, XP002960716
- MATA J.F. ET AL.: 'Role of the human concentrative nucleoside transporter (hCNT1) in the cytotoxic action of 5(prime)-deoxy-5-fluorouridine, an active intermediate metabolite of capecitabine, a novel oral anticancer drug' MOL. PHARMACOL. vol. 59, no. 6, June 2001, pages 1542 - 1548, XP002960717
- METZGER R. ET AL.: 'ERCC1 mRNA levels complement thymidylate synthase mRNA levels in predicting response and survival for gastric cancer patients receiving combination cisplatin and fluorouracil chemotherapy' J. CLIN. ONCOL. vol. 16, no. 1, 1998, pages 309 - 316, XP001118953
- DUSSEAU C. ET AL.: 'Analysis of uracil DNA glycosylase in human colorectal cancer' INT. J. ONCOL. vol. 18, no. 2, February 2001, pages 393 - 399, XP002960718
- ZEMBUTSU H. ET AL.: 'Genome-wide cDNA microarray screening to correlate gene expression profiles with sensitivity of 85 human cancer xenografts to anticancer drugs' CANCER RES. vol. 62, no. 2, January 2002, pages 518 - 527, XP002224797
- KIHARA C. ET AL.: 'Prediction of sensitivity of esophageal tumors to adjuvant chemotherapy by cDNA microarray analysis of gene-expression profiles' CANCER RES. vol. 61, no. 17, September 2001, pages 6474 - 6479, XP002960719
- WANG W. ET AL.: 'Pharmacogenomic dissection of resistance to thymidylate synthase inhibitors' CANCER RES. vol. 61, no. 14, July 2001, pages 5505 - 5510, XP002960720
- ROMAIN S. ET AL: 'DNA-synthesis enzyme activity: A biological tool useful for predicting anti-metabolic drug sensitivity in breast cancer? ' INTERNATIONAL JOURNAL OF CANCER vol. 74, no. 2, 22 April 1997, pages 156 - 161
- KIMURA H. ET AL: 'Apoptpsis, cell proliferation and expression of oncogenes in gastric carcinomas induced by preoperative administration of 5-fluorouracil' ONCOLOGY REPORTS vol. 7, no. 5, September 2000, pages 971 - 976, XP008098240

SEQUENCE LISTING

<110> TAIHO PHARMACEUTICAL CO. , LTD

<120> DNA Arrays for Measuring Sensitivity to Anticancer Agent

<130> TH0030

<140>
<141>

<150> JP P2001-204448
<151> 2001-07-05

<150> JP P2001-239181
<151> 2001-08-07

<160> 54

<170> PatentIn Ver. 2.1

<210> 1
<211> 16
<212> DNA
<213> Artificial Sequence

<220>
<223> Description of Artificial Sequence: Designed universal primer(pCR-R)

<400> 1
tgctggaatt cgccct 16

<210> 2
<211> 16
<212> DNA
<213> Artificial Sequence

<220>
<223> Description of Artificial Sequence: Designed universal primer(pCR-R)

<400> 2
ctgcagaatt cgccct 16

<210> 3
<211> 368
<212> DNA
<213> Artificial Sequence

<220>
<223> Description of Artificial Sequence: Designed fragment based on TS

<400> 3

ggatgccgag gtaaaagttc tttttgctct aaaagaaaaa ggaactaggt caaaaatctg 60

tccgtgacct atcagttatt aatttttaag gatgttgcca ctggcaaatg taactgtgcc 120

agttctttcc ataataaaag gctttgagtt aactcactga gggtatctga caatgctgag 180

gttatgaaca aagtgaggag aatgaaatgt atgtgctctt agcaaaaaca tgtatgtgca 240

tttcaatccc acgtacttat aaagaaggtt ggtgaatttc acaagctatt tttggaatat 300

ttttagaata ttttaagaat ttcacaagct attccctcaa atctgaggga gctgagtaac 360

accatcga 368

<210> 4
<211> 388
<212> DNA
<213> Artificial Sequence

<220>
<223> Description of Artificial Sequence: Designed fragment based on DPD

<400> 4

cgccgagtgt tcatcgtctt cagaaaaggc tttgttaata taagagctgt ccctgaggag 60

atggagcttg ctaaggaaga aaagtgtgaa tttctgccat tcctgtcccc acggaaggtt 120

atagtaaaag gtgggagaat tgttgctatg cagtttgttc ggacagagca agatgaaact 180

ggaaaatgga atgaagatga agatcagatg gtccatctga aagccgatgt ggtcatcagt 240

gcctttggtt cagttctgag tgatcctaaa gtaaaagaag ccttgagccc tataaaattt 300

aacagatggg gtctcccaga agtagatcca gaaactatgc aaactagtga agcatgggta 360

tttgcaggtg gtgatgtcgt tggtttgg 388

<210> 5
<211> 364
<212> DNA
<213> Artificial Sequence

<220>
<223> Description of Artificial Sequence: Designed fragment based on OPRT

<400> 5

ggttttattt ctggctcccg agtaagcatg aaaccagaat ttcttcactt gactccagga 60

gttcagttgg aagcaggagg agataatctt ggccaacagt acaatagccc acaagaagtt 120

attggcaaac gaggttccga tatcatcatt gtaggtcgtg gcataatctc agcagctgat 180

cgtctggaag cagcagagat gtacagaaaa gctgcttggg aagcgtattt gagtagactt 240

ggtgtttgag tgcttcagat acatttttca gatacaatgt gaagacattg aagatatgtg 300

gtcctcctga aagtcactgg ctggaaataa tccaattatt cctgcttgga ttcttccaca 360

gggc 364

<210> 6
<211> 308
<212> DNA
<213> Artificial Sequence

<220>
<223> Description of Artificial Sequence: Designed fragment based on TP

<400> 6

gacaaggtca gcctggtcct cgcacctgcc ctggcggcat gtggctgcaa ggtgccaatg 60
atcagcggac gtggtctggg gcacacagga ggcaccttgg ataagctgga gtctattcct 120
ggattcaatg tcatccagag cccagagcag atgcaagtgc tgctggacca ggcgggctgc 180
tgtatcgtgg gtcagagtga gcagctggtt cctgcggacg gaatcctata tgcagccaga 240
gatgtgacag ccaccgtgga cagcctgcca ctcatcacag cctccattct cagtaagaaa 300
ctcgtgga 308

<210> 7
<211> 314
<212> DNA
<213> Artificial Sequence

<220>
<223> Description of Artificial Sequence: Designed fragment based on TK1

<400> 7

aagtaccact ccgtgtgtcg gctctgctac ttcaagaagg cctcaggcca gcctgccggg 60
ccggacaaca aagagaactg cccagtgcca ggaaagccag gggaagccgt ggctgccagg 120
aagctctttg ccccacagca gattctgcaa tgcagccctg ccaactgagg gacctgcaag 180
ggccgcccgc tcccttcctg ccactgccgc ctactggacg ctgccctgca tgctgcccag 240
ccactccagg aggaagtcgg gaggcgtgga gggtgaccac accttggcct tctgggaact 300
ctcctttgtg tggc 314

<210> 8
<211> 342
<212> DNA
<213> Artificial Sequence

<220>
<223> Description of Artificial Sequence: Designed fragment based on RRM1

<400> 8

aaataaacgc catcgcccca ttggaattgg ggtacaaggt ctggcagatg cttttatcct 60
gatgagatac ccttttgaga gtgcagaagc ccagttactg aataagcaga tctttgaaac 120
tatttattat ggtgctctgg aagccagctg tgaccttgcc aaggagcagg gcccatacga 180
aacctatgag ggctctccag ttagcaaagg aattcttcag tatgatatgt ggaatgttac 240
tcctacagac ctatgggact ggaaggttct caaggagaag attgcaaagt atggtataag 300
aaacagttta cttattgccc cgatgcctac agcttccact gc 342

<210> 9
<211> 258
<212> DNA
<213> Artificial Sequence

<220>
<223> Description of Artificial Sequence: Designed fragment based on RRM2

<400> 9

gggagccaat tcacaattca ctaagtgact aaagtaagtt aaacttgtgt agactaagca 60
tgtaattttt aagttttatt ttaatgaatt aaaatatttg ttaaccaact ttaaagtcag 120
tcctgtgtat acctagatat tagtcagttg gtgccagata gaagacaggt tgtgttttta 180
tcctgtggct tgtgtagtgt cctgggattc tctgcccccct ctgagtagag tgttgtggga 240
taaaggaatc tctcaggg 258

<210> 10
<211> 549
<212> DNA
<213> Artificial Sequence

<220>
<223> Description of Artificial Sequence: Designed fragment based on UCK2

<400> 10

cccaacggcg gcgagccctt ccttataggc gtcagcgggg gaacagctag cggcaagtct 60
tccgtgtgtg ctaagatcgt gcagctcctg gggcagaatg aggtggacta tcgccagaag 120
caggtggtca tcctgagcca ggatagcttc taccgtgtcc ttacctcgga gcagaaggcc 180
aaagccctga agggccagtt caactttgac cacccggatg cctttgacaa tgaactcatt 240
ctcaaaacac tcaaagaaat cactgaaggg aaaacagtcc agatccccgt gtatgacttt 300
gtctcccatt cccggaagga ggagacagtt actgtctatc ccgcagacgt ggtgctcttt 360
gaagggatcc tggccttcta ctcccaggag gtacgagacc tgttccagat gaagcttttt 420
gtggatacag atgcggacac ccggctctca cgcagagtat taagggacat cagcgagaga 480
ggcagggatc ttgagcagat tttatctcag tacattacgt tcgtcaagcc tgcctttgag 540
gaattctgc 549

<210> 11
<211> 384
<212> DNA
<213> Artificial Sequence

<220>
<223> Description of Artificial Sequence: Designed fragment based on UP

<400> 11

ttctgcagag ctgagcgagt tcaccacagt ggtggggaac accatgtgca ccttggactt 60
ctatgaaggg caaggccgtc tggatggggc tctctgctcc tacacggaga aggacaagca 120
ggcgtatctg gaggcagcct atgcagccgg cgtccgcaat atcgagatgg agtcctcggt 180
gtttgccgcc atgtgcagcg cctgcggcct ccaagcggcc gtggtgtgtg tcaccctcct 240
gaaccgcctg gaaggggacc agatcagcag ccctcgcaat gtgctcagcg agtaccagca 300
gaggccgcag cggctggtga gctacttcat caagaagaaa ctgagcaagg cctgagcgct 360
gccctgcacc tccgcagacc tgct 384

<210> 12
<211> 302
<212> DNA
<213> Artificial Sequence

<220>
<223> Description of Artificial Sequence: Designed fragment based on CDA

<400> 12

ggcatctgtg ctgaacggac cgctatccag aaggccgtct cagaagggta caaggatttc 60

agggcaattg ctatcgccag tgacatgcaa gatgatttta tctctccatg tggggcctgc 120

aggcaagtca tgagagagtt tggcaccaac tggcccgtgt acatgaccaa gccggatggt 180

acgtatattg tcatgacggt ccaggagctg ctgccctcct cctttgggcc tgaggacctg 240

cagaagactc agtgacagcc agagaatgcc cactgcctgt aacagccacc tggagaactt 300

ca 302

<210> 13
<211> 274
<212> DNA
<213> Artificial Sequence

<220>
<223> Description of Artificial Sequence: Designed fragment based on NT5

<400> 13

ttctaaacct gcacttgtcc ctctccagca agaggctagc actgaattca ttctactcat 60

actacacacc cagttatgga atgtccagag ttctcgaaga aaataaatga ctttaggaag 120

aggtatacat tttttaagtc gctctgcctc caaatctgaa cagtcactgt aaatcattct 180

taagcccaga tatgagaact tctgctggaa agtgggaccc tctgagtggg tggtcagaaa 240

atacccatgc tgatgaaatg acctatgccc aaag 274

<210> 14
<211> 254
<212> DNA
<213> Artificial Sequence

<220>
<223> Description of Artificial Sequence: Designed fragment based on IMPD

<400> 14

tcgtgcccta cctcatagca ggcatccaac acggctgcca ggatatcggg gcccgcagcc 60

tgtctgtcct tcggtccatg atgtactcag gagagctcaa gtttgagaag cggaccatgt 120

cgccccagat tgagggtggt gtccatggcc tgcactctta cgaaaagcgg ctgtactgag 180

gacagcggtg gaggccgagg tggtggaggg gatgcacccc agtgtccact tttgggcaca 240

ggctccctcc ataa 254

<210> 15
<211> 304
<212> DNA
<213> Artificial Sequence

<220>
<223> Description of Artificial Sequence: Designed fragment based on MTHFD1

<400> 15

tcctggctct caccacttct ctagaagaca tgagagagag actgggcaaa atggtggtgg 60

catccagtaa gaaaggagag cccgtcagtg ccgaagatct gggggtgagt ggtgcactga 120

cagtgcttat gaaggacgca atcaagccca atctcatgca gacactggag ggcactccag 180

tgtttgtcca tgctggcccg tttgccaaca tcgcacatgg caattcctcc atcattgcag 240

accagatcgc actcaagctt gttggcccag aagggtttgt agtgacggaa gcaggatttg 300

gagc 304

<210> 16
<211> 331
<212> DNA
<213> Artificial Sequence

<220>
<223> Description of Artificial Sequence: Designed fragment based on RP2

<400> 16

aagagtggac ttctcggccc gtactgtcat cacccccgac cccaacctct ccattgacca 60

ggttggcgtg ccccgctcca ttgctgccaa catgaccttt gcggagattg tcaccccctt 120

caacattgac agacttcaag aactagtgcg cagggggaac agtcagtacc caggcgccaa 180

gtacatcatc cgagacaatg gtgatcgcat tgacttgcgt ttccacccca agcccagtga 240

ccttcacctg cagaccggct ataaggtgga acggcacatg tgtgatgggg acattgttat 300

cttcaaccgg cagccaactc tgcacaaaat g 331

<210> 17
<211> 285
<212> DNA
<213> Artificial Sequence

<220>
<223> Description of Artificial Sequence: Designed fragment based on UMPK

<400> 17

tctaaacctg aaagcatcct tgaaatcatg cttgaatatt gctttgatag ctgctatcat 60

gaccccttttt taaggcaatt ctaatctttc ataactacat ctcaattagt ggctggaaag 120

tacatggtaa aacaaagtaa attttttat gttcttttt tggtcacagg agtagacagt 180

gaattcaggt ttaacttcac cttagttatg gtgctcacca aacgaagggt atcagctatt 240

tttttttaaa ttcaaaaaga atatcccttt tatagtttgt gcctt 285

<210> 18
<211> 265
<212> DNA
<213> Artificial Sequence

<220>
<223> Description of Artificial Sequence: Designed fragment based on CTPS

<400> 18

ccatcggtca ccttgtttct caactacctc gcatcattgc agatcgtagc gcgttgcctg 60
tcgctttccc ttggatacct agaccgttat aaagtgtgcc acatggactt accgagcatg 120
gagagaggat tttagctagg atttgaacac ttggtgctgg gaacctcagg gtattgcttg 180
ccactaagcc atgaaaccag agacaaaatc tctatactgc cctgagttgg ggggaattct 240
cagtgccaac tgtggctggt cctca 265

<210> 19
<211> 368
<212> DNA
<213> Artificial Sequence

<220>
<223> Description of Artificial Sequence: Designed fragment based on DCD

<400> 19

cagggtggtg gcacattatc cctctggggg gtggggacgc ctgttgtttt ggctcaattt 60
gggtttgttg gtcacatgga gctcttccat ttcgtttagc tgaataatga gttgttccta 120
gaggagacag cctgtctctc cttgttgccc ccaaagccca tgccctgccg tggtggcagc 180
tggggctgtg gatgggaggg gtccccaaca tggatgtgtt gcccctcctc cgcatgccaa 240
cgcagttcat gtacaaggcc cctctgcaac tggagagaaa attaattcct atcccgtgag 300
tggattgtga gaaattccac ccacgtggag acagcttact gcagcactgt tggtgttcgg 360
agctcttc 368

<210> 20
<211> 422
<212> DNA
<213> Artificial Sequence

<220>
<223> Description of Artificial Sequence: Designed fragment based on DCK

<400> 20

gatctgtgta tagtgacagg tatatttttg catctaattt gtatgaatct gaatgcatga 60

atgagacaga gtggacaatt tatcaagact ggcatgactg gatgaataac caatttggcc 120

aaagccttga attggatgga atcatttatc ttcaagccac tccagagaca tgcttacata 180

gaatatattt acggggaaga aatgaagagc aaggcattcc tcttgaatat ttagagaagc 240

ttcattataa acatgaaagc tggctcctgc ataggacact gaaaaccaac ttcgattatc 300

ttcaagaggt gcctatctta acactggatg ttaatgaaga ctttaaagac aaatatgaaa 360

gtctggttga aaaggtcaaa gagtttttga gtactttgtg atcttgctga agactacagg 420

ca 422

<210> 21
<211> 322
<212> DNA
<213> Artificial Sequence

<220>
<223> Description of Artificial Sequence: Designed fragment based on PRPS

<400> 21

attcagcaga agacccggct tgctccagtg tagctttcta catcccacat caggtatatt 60

agagcttatc cgaactgggg aaagacggat tgagattaac tgctgggacc tcctacctgc 120

attatctcat tctggcttcc ttgataattc tgtgggcctt gcagctttaa ctatagctca 180

gctgctgcaa gatttcagac ttttgaggat gttgtgtgag ggtgtttgac tgtgactggg 240

gaagctcaga ctactttgta tgtgaatgct tcagggtttt ctttgttgag aacaactagc 300

aacaaaggca acccatgtgt ga 322

<210> 22
<211> 391
<212> DNA
<213> Artificial Sequence

<220>
<223> Description of Artificial Sequence: Designed fragment based on HPRT1

<400> 22

gccatctgct tagtagagct ttttgcatgt atcttctaag aattttatct gttttgtact 60
ttagaaatgt cagttgctgc attcctaaac tgtttatttg cactatgagc ctatagacta 120
tcagttccct ttgggcggat tgttgtttaa cttgtaaatg aaaaaattct cttaaaccac 180
agcactattg agtgaaacat tgaactcata tctgtaagaa ataaagagaa gatatattag 240
tttttaatt ggtattttaa tttttatata tgcaggaaag aatagaagtg attgaatatt 300
gttaattata ccaccgtgtg ttagaaaagt aagaagcagt caattttcac atcaaagaca 360
gcatctaaga agttttgttc tgtcctggaa t 391

<210> 23
<211> 220
<212> DNA
<213> Artificial Sequence

<220>
<223> Description of Artificial Sequence: Designed fragment based on FPGS

<400> 23

cgcatgctca ataccctgca gaccaatgcc ggctacctgg agcaggtgaa gcgccagcgg 60
ggtgaccctc agacacagtt ggaagccatg gaactgtacc tggcacggag tgggctgcag 120
gtggaggact tggaccggct gaacatcatc cacgtcactg ggacgaaggg gaagggctcc 180
acctgtgcct tcacggaatg tatcctccga agctatggcc 220

<210> 24
<211> 364
<212> DNA
<213> Artificial Sequence

<220>
<223> Description of Artificial Sequence: Designed fragment based on NDKA

<400> 24

cggtagttgc catggtctgg gaggggctga atgtggtgaa gacgggccga gtcatgctcg 60

gggagaccaa ccctgcagac tccaagcctg ggaccatccg tggagacttc tgcatacaag 120

ttggcaggaa cattatacat ggcagtgatt ctgtggagag tgcagagaag gagatcggct 180

tgtggtttca ccctgaggaa ctggtagatt acacgagctg tgctcagaac tggatctatg 240

aatgacagga gggcagacca cattgctttt cacatccatt tcccctcctt cccatgggca 300

gaggaccagg ctgtaggaaa tctagttatt tacaggaact tcatcataat ttggagggaa 360

gctc 364

<210> 25
<211> 372
<212> DNA
<213> Artificial Sequence

<220>
<223> Description of Artificial Sequence: Designed fragment based on NDKB

<400> 25

acctgaaaga ccgaccattc ttccctgggc tggtgaagta catgaactca gggccggttg 60

tggccatggt ctgggagggg ctgaacgtgg tgaagacagg ccgagtgatg cttggggaga 120

ccaatccagc agattcaaag ccaggcacca ttcgtgggga cttctgcatt caggttggca 180

ggaacatcat tcatggcagt gattcagtaa aaagtgctga aaaagaaatc agcctatggt 240

ttaagcctga agaactggtt gactacaagt cttgtgctca tgactgggtc tatgaataag 300

aggtggacac aacagcagtc tccttcagca cggcgtggtg tgtccctgga cacagctctt 360

cattccattg ac 372

<210> 26
<211> 406
<212> DNA
<213> Artificial Sequence

<220>
<223> Description of Artificial Sequence: Designed fragment based on APRT

<400> 26

acatctcgcc cgtcctgaag gaccccgcct ccttccgcgc cgccatcggc ctcctggcgc 60

gacacctgaa ggcgacccac gggggccgca tcgactacat cgcaggccta gactcccgag 120

gcttcctctt tggcccctcc ctggcccagg agcttggact gggctgcgtg ctcatccgaa 180

agcgggggaa gctgccaggc cccactctgt gggcctccta ttccctggag tacgggaagg 240

ctgagctgga gattcagaaa gacgccctgg agccaggaca gagggtggtc gtcgtggatg 300

atctgctggc cactggtgga accatgaacg ctgcctgtga gctgctgggc cgcctgcagg 360

ctgaggtcct ggagtgcgtg agcctggtgg agctgacctc gcttaa 406

<210> 27
<211> 369
<212> DNA
<213> Artificial Sequence

<220>
<223> Description of Artificial Sequence: Designed fragment based on AK

<400> 27

ctgcaccgtt tattagccag ttctacaagg aatcattgat gaaagttatg ccttatgttg 60

atatactttt tggaaatgag acagaagctg ccacttttgc tagagagcaa ggctttgaga 120

ctaaagacat taaagagata gccaaaaaga cacaagccct gccaaagatg aactcaaaga 180

ggcagcgaat cgtgatcttc acccaaggga gagatgacac tataatggct acagaaagtg 240

aagtcactgc ttttgctgtc ttggatcaag accagagaga aattattgat accaatggag 300

ctggagatgc atttgttgga ggttttctgt ctcaactggt ctctgacaag ccactgactg 360

aatgtatcc 369

<210> 28
<211> 317
<212> DNA
<213> Artificial Sequence

<220>
<223> Description of Artificial Sequence: Designed fragment based on ERCC1

<400> 28

tgatacccct cgacgaggat gaggtccctc ctggagtggc caagccctta ttccgatcta 60
cacagagcct tcccactgtg gacacctcgg cccaggcggc ccctcagacc tacgccgaat 120
atgccatctc acagcctctg gaaggggctg gggccacgtg ccccacaggg tcagagcccc 180

tggcaggaga gacgcccaac caggccctga aacccggggc aaaatccaac agcatcattg 240
tgagccctcg gcagaggggc aatcccgtac tgaagttcgt gcgcaacgtg ccctgggaat 300
ttggcgacgt aattccc 317

<210> 29
<211> 391
<212> DNA
<213> Artificial Sequence

<220>
<223> Description of Artificial Sequence: Designed fragment based on UDG

<400> 29

atctggccca gaaattaggg ctcaatttcc tgattgtagt agaggttaag attgctgtga 60
gctttatcag ataagagacc gagagaagta agctgggtct tgttattcct tgggtgttgg 120
tggaataagc agtggaattt gaacaaggaa gaggagaaaa gggaattttg tctttatggg 180
gtggggtgat tttctcctag ggttatgtcc agttggggtt tttaaggcag cacagactgc 240
caagtactgt ttttttaac cgactgaaat cactttggga tattttttcc tgcaacactg 300
gaaagtttta gtttttaag aagtactcat gcagatatat atatatatat ttttcccagt 360
cctttttta agagacggtc tttattgggt c 391

<210> 30
<211> 410
<212> DNA
<213> Artificial Sequence

<220>
<223> Description of Artificial Sequence: Designed fragment based on PARP

<400> 30

aaggcgaatg ccagcgttac aagcccttta agcagcttca taaccgaaga ttgctgtggc 60
acgggtccag gaccaccaac tttgctggga tcctgtccca gggtcttcgg atagccccgc 120
ctgaagcgcc cgtgacaggc tacatgtttg gtaaagggat ctatttcgct gacatggtct 180
ccaagagtgc caactactac catacgtctc agggagaccc aataggctta atcctgttgg 240
gagaagttgc ccttggaaac atgtatgaac tgaagcacgc ttcacatatc agcaggttac 300
ccaagggcaa gcacagtgtc aaaggtttgg gcaaaactac ccctgatcct tcagctaaca 360
ttagtctgga tggtgtagac gttcctcttg ggaccgggat ttcatctggt 410

<210> 31
<211> 309
<212> DNA
<213> Artificial Sequence

<220>
<223> Description of Artificial Sequence: Designed fragment based on LIG1

<400> 31

actggcagtg cttccacagc caagaagata gacatcatca aaggcctctt tgtggcctgc 60
cgccactcag aagcccggtt catcgctagg tccctgagcg gacggctgcg ccttgggctg 120
gcagagcagt cggtgctggc tgccctctcc caggcagtga gcctcacgcc cccgggccaa 180
gaattcccac cagccatggt ggatgctggg aagggcaaga cagcagaggc cagaaagacg 240
tggctggagg agcaaggcat gatcctgaag cagacgttct gcgaggttcc cgacctggac 300
cgaattatc 309

<210> 32
<211> 355
<212> DNA
<213> Artificial Sequence

<220>
<223> Description of Artificial Sequence: Designed fragment based on LIG3

<400> 32

caaggtggcc cactttaagg actacattcc ccaggctttt cctgggggcc acagcatgat 60
cttggattct gaagtgcttc tgattgacaa caagacaggc aaaccactgc cctttgggac 120
tctgggagta cacaagaaag cagccttcca ggatgctaat gtctgcctgt ttgtttttga 180
ttgtatctac tttaatgatg tcagcttgat ggacagacct ctgtgtgagc ggcggaagtt 240
tcttcatgac aacatggttg aaattccaaa ccggatcatg ttctcagaaa tgaagcgagt 300
cacaaaagct ttggacttgg ctgacatgat aacccgggtg atccaggagg gattg 355

<210> 33
<211> 411
<212> DNA
<213> Artificial Sequence

<220>
<223> Description of Artificial Sequence: Designed fragment based on LIG4

<400> 33

ggcatctggt aagctcgcat ctaaacacct ttatataggt ggtgatgatg aaccacaaga 60
aaaaaagcgg aaagctgccc caaagatgaa gaaagttatt ggaattattg agcacttaaa 120
agcacctaac cttactaacg ttaacaaaat ttctaatata tttgaagatg tagagttttg 180
tgttatgagt ggaacagata gccagccaaa gcctgacctg gagaacagaa ttgcagaatt 240
tggtggttat atagtacaaa atccaggccc agacacgtac tgtgtaattg cagggtctga 300
gaacatcaga gtgaaaaaca taattttgtc aaataaacat gatgttgtca agcctgcatg 360
gcttttagaa tgttttaaga ccaaaagctt tgtaccatgg cagcctcgct t 411

<210> 34
<211> 335
<212> DNA
<213> Artificial Sequence

<220>
<223> Description of Artificial Sequence: Designed fragment based on POLB

<400> 34

cgccatgagc aaacggaagg cgccgcagga gactctcaac gggggaatca ccgacatgct 60

cacagaactc gcaaactttg agaagaacgt gagccaagct atccacaagt acaatgctta 120

cagaaaagca gcatctgtta tagcaaaata cccacacaaa ataaagagtg gagctgaagc 180

taagaaattg cctggagtag gaacaaaaat tgctgaaaag attgatgagt tttagcaac 240

tggaaaatta cgtaaactgg aaaagattcg gcaggatgat acgagttcat ccatcaattt 300

cctgactcga gttagtggca ttggtccatc tgctg 335

<210> 35
<211> 262
<212> DNA
<213> Artificial Sequence

<220>
<223> Description of Artificial Sequence: Designed fragment based on POLD

<400> 35

ccaccttcat ccgtatcatg gaccccgacg tgatcaccgg ttacaacatc cagaacttcg 60

accttccgta cctcatctct cgggcccaga ccctcaaggt acaaacattc cctttcctgg 120

gccgtgtggc cggcctttgc tccaacatcc gggactcttc attccagtcc aagcagacgg 180

gccggcggga caccaaggtt gtcagcatgg tgggccgcgt gcagatggac atgctgcagg 240

tgctgctgcg ggagtacaag ct 262

<210> 36
<211> 368
<212> DNA
<213> Artificial Sequence

<220>
<223> Description of Artificial Sequence: Designed fragment based on XRCC1

<400> 36

ctgtcgccat ctgttcccaa gagacctaaa ttgccagctc caactcgtac cccagccaca 60
gccccagtcc ctgcccgagc acagggggca gtgacaggca aaccccgagg agaaggcacc 120
gagcccagac gaccccgagc tggcccagag gagctgggga agatccttca gggtgtggta 180
gtggtgctga gtggcttcca gaaccccttc cgctccgagc tgcgagataa ggccctagag 240
cttggggcca agtatcggcc agactggacc cgggacagca cgcacctcat ctgtgccttt 300
gccaacaccc ccaagtacag ccaggtccta ggcctgggag gccgcatcgt gcgtaaggag 360
tgggtgct 368

<210> 37
<211> 324
<212> DNA
<213> Artificial Sequence

<220>
<223> Description of Artificial Sequence: Designed fragment based on TOP1

<400> 37

tatttcaaag cccagacgga agctcggaaa cagatgagca aggaagagaa actgaaaatc 60
aaagaggaga atgaaaaatt actgaaagaa tatggattct gtattatgga taaccacaaa 120
gagaggattg ctaacttcaa gatagagcct cctggacttt tccgtggccg cggcaaccac 180
cccaagatgg gcatgctgaa gagacgaatc atgcccgagg atataatcat caactgtagc 240
aaagatgcca aggttccttc tcctcctcca ggacataagt ggaaagaagt ccggcatgat 300
aacaaggtta cttggctggt ttcc 324

<210> 38
<211> 265
<212> DNA
<213> Artificial Sequence

<220>
<223> Description of Artificial Sequence: Designed fragment based on MDR1

<400> 38

tcagttaccc atctcgaaaa gaagttaaga tcttgaaggg tctgaacctg aaggtgcaga 60
gtgggcagac ggtggccctg gttggaaaca gtggctgtgg gaagagcaca acagtccagc 120

tgatgcagag gctctatgac cccacagagg ggatggtcag tgttgatgga caggatatta 180

ggaccataaa tgtaaggttt ctacgggaaa tcattggtgt ggtgagtcag gaacctgtat 240

tgtttgccac cacgatagct gaaaa 265

<210> 39
<211> 256
<212> DNA
<213> Artificial Sequence

<220>
<223> Description of Artificial Sequence: Designed fragment based on ENT1

<400> 39

tcagccaggg aaaaccgaga acaccatcac catgacaacc agtcaccagc ctcaggacag 60

atacaaagct gtctggctta tcttcttcat gctgggtctg ggaacgctgc tcccgtggaa 120

ttttttcatg acggccactc agtatttcac aaaccgcctg gacatgtccc agaatgtgtc 180

cttggtcact gctgaactga gcaaggacgc ccaggcgtca gccgcccctg cagcaccctt 240

gcctgagcgg aactct 256

<210> 40
<211> 399
<212> DNA
<213> Artificial Sequence

<220>
<223> Description of Artificial Sequence: Designed fragment based on MRP1

<400> 40

cctgtttgcg gtgatctcca ggcacagcct cagtgctggc ttggtgggcc tctcagtgtc 60

ttactcattg caggtcacca cgtacttgaa ctggctggtt cggatgtcat ctgaaatgga 120

aaccaacatc gtggccgtgg agaggctcaa ggagtattca gagactgaga aggaggcgcc 180

ctggcaaatc caggagacag ctccgcccag cagctggccc caggtgggcc gagtggaatt 240

ccggaactac tgcctgcgct accgagagga cctggacttc gttctcaggc acatcaatgt 300

cacgatcaat gggggagaaa aggtcggcat cgtggggcgg acgggagctg ggaagtcgtc 360

cctgaccctg ggcttatttc ggatcaacga gtctgccga 399

<210> 41
<211> 353
<212> DNA
<213> Artificial Sequence

<220>
<223> Description of Artificial Sequence: Designed fragment based on TOP2A

<400> 41

gtgctttttg accacgtagg ctgtttaaag aaatatgaca cggtgttgga tattctaaga 60

gactttttg aactcagact taaatattat ggattaagaa aagaatggct cctaggaatg 120

cttggtgctg aatctgctaa actgaataat caggctcgct ttatcttaga gaaaatagat 180

ggcaaaataa tcattgaaaa taagcctaag aaagaattaa ttaaagttct gattcagagg 240

ggatatgatt cggatcctgt gaaggcctgg aaagaagccc agcaaaaggt tccagatgaa 300

gaagaaaatg aagagagtga caacgaaaag gaaactgaaa agagtgactc cgt 353

<210> 42
<211> 374
<212> DNA
<213> Artificial Sequence

<220>
<223> Description of Artificial Sequence: Designed fragment based on TOP2B

<400> 42

ttacgtaagg agtggcttgt gggaatgttg ggagcagaat ctacaaagct taacaatcaa 60

gcccgtttca ttttagagaa gatacaaggg aaaattacta tagagaatag gtcaaagaaa 120

gatttgattc aaatgttagt ccagagaggt tatgaatctg acccagtgaa agcctggaaa 180

gaagcacaag aaaaggcagc agaagaggat gaaacacaaa accagcatga tgatagttcc 240

tccgattcag gaactccttc aggcccagat tttaattata ttttaaatat gtctctgtgg 300

tctcttacta aagaaaaagt tgaagaactg attaaacaga gagatgcaaa agggcgagag 360

gtcaatgatc ttaa 374

<210> 43
<211> 212
<212> DNA
<213> Artificial Sequence

<220>
<223> Description of Artificial Sequence: Designed fragment based on Hsp27

<400> 43

ctacagccgc gcgctcagcc ggcaactcag cagcggggtc tcggagatcc ggcacactgc 60
ggaccgctgg cgcgtgtccc tggatgtcaa ccacttcgcc ccggacgagc tgacggtcaa 120
gaccaaggat ggcgtggtgg agatcaccgg caagcacgag gagcggcagg acgagcatgg 180
ctacatctcc cggtgcttca cgcggaaata ca 212

<210> 44
<211> 323
<212> DNA
<213> Artificial Sequence

<220>
<223> Description of Artificial Sequence: Designed fragment based on ENT2

<400> 44

tgaccagagg gttcagagtg ggaggcaggg ccagcccagg ccaggagcgc ctcatcttcc 60
caggcctcag ccacccaggg taaaaggtgc cagggaagtt gtgggcacct gagaggagga 120
acagatgtgg aggacctgag ggtgctcaaa gggccaggct cagcctcaag cagtgttttc 180
attgccaaca cttactgtac ccactccgca gagccccgct gggcctgggc cccagggcca 240
cagctagcct gcatgtgtgt actgcacttt acagtttgca aagctcttcc atacccactc 300
tctcaccgaa gcctaattga ggc 323

<210> 45
<211> 296
<212> DNA
<213> Artificial Sequence

<220>
<223> Description of Artificial Sequence: Designed fragment based on E2F1

<400> 45

cgatgttttc ctgtgccctg aggagaccgt aggtgggatc agccctggga agaccccatc 60
ccaggaggtc acttctgagg aggagaacag ggccactgac tctgccacca tagtgtcacc 120
accaccatca tctcccccct catccctcac cacagatccc agccagtctc tactcagcct 180
ggagcaagaa ccgctgttgt cccggatggg cagcctgcgg gctcccgtgg acgaggaccg 240
cctgtccccg ctggtggcgg ccgactcgct cctggagcat gtgcgggagg acttct 296

<210> 46
<211> 335
<212> DNA
<213> Artificial Sequence

<220>
<223> Description of Artificial Sequence: Designed fragment based on p53

<400> 46

gctcagatag cgatggtctg gcccctcctc agcatcttat ccgagtggaa ggaaatttgc 60
gtgtggagta tttggatgac agaaacactt ttcgacatag tgtggtggtg ccctatgagc 120
cgcctgaggt tggctctgac tgtaccacca tccactacaa ctacatgtgt aacagttcct 180
gcatgggcgg catgaaccgg aggcccatcc tcaccatcat cacactggaa gactccagtg 240
gtaatctact gggacggaac agctttgagg tgcgtgtttg tgcctgtcct gggagagacc 300
ggcgcacaga ggaagagaat ctccgcaaga aaggg 335

<210> 47
<211> 279
<212> DNA
<213> Artificial Sequence

<220>
<223> Description of Artificial Sequence: Designed fragment based on VEGFB

<400> 47

cagaggaaag tggtgtcatg gatagatgtg tatactcgcg ctacctgcca gccccgggag 60

gtggtggtgc ccttgactgt ggagctcatg ggcaccgtgg ccaaacagct ggtgcccagc 120

tgcgtgactg tgcagcgctg tggtggctgc tgccctgacg atggcctgga gtgtgtgccc 180

actgggcagc accaagtccg gatgcagatc ctcatgatcc ggtacccgag cagtcagctg 240

ggggagatgt ccctggaaga acacagccag tgtgaatgc 279

<210> 48
<211> 368
<212> DNA
<213> Artificial Sequence

<220>
<223> Description of Artificial Sequence: Designed fragment based on ITGA3

<400> 48

gctgtatccc acggagatca ccgtccatgg caatgggtcc tggccctgcc gaccacctgg 60

agaccttatc aaccctctca acctcactct ttctgaccct ggggacaggc catcatcccc 120

acagcgcagg cgccgacagc tggatccagg gggaggccag ggccccccac ctgtcactct 180

ggctgctgcc aaaaaagcca agtctgagac tgtgctgacc tgtgccacag ggcgtgccca 240

ctgtgtgtgg ctagagtgcc ccatccctga tgcccccgtt gtcaccaacg tgactgtgaa 300

ggcacgagtg tggaacagca ccttcatcga ggattacaga gactttgacc gagtccgggt 360

aaatggct 368

<210> 49
<211> 270
<212> DNA
<213> Artificial Sequence

<220>
<223> Description of Artificial Sequence: Designed fragment based on SOD2

<400> 49

atctgggctc caggcagaag cacagcctcc ccgacctgcc ctacgactac ggcgccctgg 60

aacctcacat caacgcgcag atcatgcagc tgcaccacag caagcaccac gcggcctacg 120

tgaacaacct gaacgtcacc gaggagaagt accaggaggc gttggccaag ggagatgtta 180

cagcccagat agctcttcag cctgcactga agttcaatgg tggtggtcat atcaatcata 240

gcattttctg gacaaacctc agccctaacg 270

<210> 50
<211> 260
<212> DNA
<213> Artificial Sequence

<220>
<223> Description of Artificial Sequence: Designed fragment based on SOD1

<400> 50

gcctagcgag ttatggcgac gaaggccgtg tgcgtgctga agggcgacgg cccagtgcag 60

ggcatcatca atttcgagca gaaggaaagt aatggaccag tgaaggtgtg gggaagcatt 120

aaaggactga ctgaaggcct gcatggattc catgttcatg agtttggaga taatacagca 180

ggctgtacca gtgcaggtcc tcactttaat cctctatcca gaaaacacgg tgggccaaag 240

gatgaagaga ggcatgttgg 260

<210> 51
<211> 316
<212> DNA
<213> Artificial Sequence

<220>
<223> Description of Artificial Sequence: Designed fragment based on PCNA

<400> 51

actccgccac catgttcgag gcgcgcctgg tccagggctc catcctcaag aaggtgttgg 60

aggcactcaa ggacctcatc aacgaggcct gctgggatat tagctccagc ggtgtaaacc 120

tgcagagcat ggactcgtcc cacgtctctt tggtgcagct caccctgcgg tctgagggct 180

tcgacaccta ccgctgcgac cgcaacctgg ccatgggcgt gaacctcacc agtatgtcca 240

aaatactaaa atgcgccggc aatgaagata tcattacact aagggccgaa gataacgcgg 300

ataccttggc gctagt 316

<210> 52
<211> 333
<212> DNA
<213> Artificial Sequence

<220>
<223> Description of Artificial Sequence: Designed fragment based on GAPDH

<400> 52

cgtcatgggt gtgaaccatg agaagtatga caacagcctc aagatcatca gcaatgcctc 60

ctgcaccacc aactgcttag cacccctggc caaggtcatc catgacaact ttggtatcgt 120

ggaaggactc atgaccacag tccatgccat cactgccacc cagaagactg tggatggccc 180

ctccgggaaa ctgtggcgtg atggccgcgg ggctctccag aacatcatcc ctgcctctac 240

tggcgctgcc aaggctgtgg gcaaggtcat ccctgagcta gacgggaagc tcactggcat 300

ggccttccgt gtccccactg ccaacgtgtc agt 333

<210> 53
<211> 264
<212> DNA
<213> Artificial Sequence

<220>
<223> Description of Artificial Sequence: Designed fragment based on ACTB

<400> 53

cattggcaat gagcggttcc gctgccctga ggcactcttc cagccttcct tcctgggcat 60

ggagtcctgt ggcatccacg aaactacctt caactccatc atgaagtgtg acgtggacat 120

ccgcaaagac ctgtacgcca acacagtgct gtctggcggc accaccatgt accctggcat 180

tgccgacagg atgcagaagg agatcactgc cctggcaccc agcacaatga agatcaagat 240

cattgctcct cctgagcgca agta 264

<210> 54
<211> 361
<212> DNA
<213> Artificial Sequence

<220>
<223> Description of Artificial Sequence: Designed fragment based on RSP9

<400> 54

tcctgggcct gaagatagag gatttcttag agagacgcct gcagacccag gtcttcaagc 60

tgggcttggc caagtccatc caccacgctc gcgtgctgat ccgccagcgc catatcaggg 120

tccgcaagca ggtggtgaac atcccgtcct tcattgtccg cctggattcc cagaagcaca 180

ttgacttctc tctgcgctct cctacggggg ttggccgccc gggccgcgtg aagaggaaga 240

atgccaagaa gggccagggt ggggctgggg ctggagacga cgaggaggag gattaagtcc 300

acctgtccct cctgggctgc tggattgtct cgttttcctg ccaaataaac aggatcagcg 360

c 361

SEQUENCE LISTING

<110> TAIHO PHARMACEUTICAL CO. , LTD

<120> DNA Arrays for Measuring Sensitivity to Anticancer Agent

<130> TH0030

<140>
<141>

<150> JP P2001-204448
<151> 2001-07-05

<150> JP P2001-239181
<151> 2001-08-07

<160> 54

<170> PatentIn Ver. 2.1

<210> 1
<211> 16
<212> DNA
<213> Artificial Sequence

<220>
<223> Description of Artificial Sequence: Designed universal primer(pCR-R)

<400> 1
tgctggaatt cgccct 16

<210> 2
<211> 16
<212> DNA
<213> Artificial Sequence

<220>
<223> Description of Artificial Sequence: Designed universal primer(pCR-R)

<400> 2
ctgcagaatt cgccct 16

<210> 3
<211> 368
<212> DNA
<213> Artificial Sequence

<220>
<223> Description of Artificial Sequence: Designed fragment based on TS

<400> 3

ggatgccgag gtaaaagttc tttttgctct aaaagaaaaa ggaactaggt caaaaatctg 60

tccgtgacct atcagttatt aatttttaag gatgttgcca ctggcaaatg taactgtgcc 120

agttctttcc ataataaaag gctttgagtt aactcactga gggtatctga caatgctgag 180

gttatgaaca aagtgaggag aatgaaatgt atgtgctctt agcaaaaaca tgtatgtgca 240

tttcaatccc acgtacttat aaagaaggtt ggtgaatttc acaagctatt tttggaatat 300

ttttagaata ttttaagaat ttcacaagct attccctcaa atctgaggga gctgagtaac 360

accatcga 368

<210> 4
<211> 388

<212> DNA
<213> Artificial Sequence

<220>
<223> Description of Artificial Sequence: Designed fragment based on DPD

<400> 4

cgccgagtgt tcatcgtctt cagaaaaggc tttgttaata taagagctgt ccctgaggag 60

atggagcttg ctaaggaaga aaagtgtgaa tttctgccat tcctgtcccc acggaaggtt 120

atagtaaaag gtgggagaat tgttgctatg cagtttgttc ggacagagca agatgaaact 180

ggaaaatgga atgaagatga agatcagatg gtccatctga aagccgatgt ggtcatcagt 240

gcctttggtt cagttctgag tgatcctaaa gtaaaagaag ccttgagccc tataaaattt 300

aacagatggg gtctcccaga agtagatcca gaaactatgc aaactagtga agcatgggta 360

tttgcaggtg gtgatgtcgt tggtttgg 388

<210> 5
<211> 364
<212> DNA
<213> Artificial Sequence

<220>
<223> Description of Artificial Sequence: Designed fragment based on OPRT

<400> 5

ggttttattt ctggctcccg agtaagcatg aaaccagaat ttcttcactt gactccagga 60

gttcagttgg aagcaggagg agataatctt ggccaacagt acaatagccc acaagaagtt 120

attggcaaac gaggttccga tatcatcatt gtaggtcgtg gcataatctc agcagctgat 180

cgtctggaag cagcagagat gtacagaaaa gctgcttggg aagcgtattt gagtagactt 240

ggtgtttgag tgcttcagat acatttttca gatacaatgt gaagacattg aagatatgtg 300

gtcctcctga aagtcactgg ctggaaataa tccaattatt cctgcttgga ttcttccaca 360

gggc 364

<210> 6
<211> 308
<212> DNA

<213> Artificial Sequence

<220>
<223> Description of Artificial Sequence: Designed fragment based on TP

<400> 6

gacaaggtca gcctggtcct cgcacctgcc ctggcggcat gtggctgcaa ggtgccaatg 60
atcagcggac gtggtctggg gcacacagga ggcaccttgg ataagctgga gtctattcct 120
ggattcaatg tcatccagag cccagagcag atgcaagtgc tgctggacca ggcgggctgc 180
tgtatcgtgg gtcagagtga gcagctggtt cctgcggacg gaatcctata tgcagccaga 240
gatgtgacag ccaccgtgga cagcctgcca ctcatcacag cctccattct cagtaagaaa 300
ctcgtgga 308

<210> 7
<211> 314
<212> DNA
<213> Artificial Sequence

<220>
<223> Description of Artificial Sequence: Designed fragment based on TK1

<400> 7

aagtaccact ccgtgtgtcg gctctgctac ttcaagaagg cctcaggcca gcctgccggg 60
ccggacaaca aagagaactg cccagtgcca ggaaagccag gggaagccgt ggctgccagg 120
aagctctttg ccccacagca gattctgcaa tgcagccctg ccaactgagg gacctgcaag 180
ggccgcccgc tcccttcctg ccactgccgc ctactggacg ctgccctgca tgctgcccag 240
ccactccagg aggaagtcgg gaggcgtgga gggtgaccac accttggcct tctgggaact 300
ctcctttgtg tggc 314

<210> 8
<211> 342
<212> DNA
<213> Artificial Sequence

<220>
<223> Description of Artificial Sequence: Designed fragment based on RRM1

<400> 8

aaataaacgc catcgcccca ttggaattgg ggtacaaggt ctggcagatg cttttatcct 60

gatgagatac ccttttgaga gtgcagaagc ccagttactg aataagcaga tctttgaaac 120

tatttattat ggtgctctgg aagccagctg tgaccttgcc aaggagcagg gcccatacga 180

aacctatgag ggctctccag ttagcaaagg aattcttcag tatgatatgt ggaatgttac 240

tcctacagac ctatgggact ggaaggttct caaggagaag attgcaaagt atggtataag 300

aaacagttta cttattgccc cgatgcctac agcttccact gc 342

<210> 9
<211> 258
<212> DNA
<213> Artificial Sequence

<220>
<223> Description of Artificial Sequence: Designed fragment based on RRM2

<400> 9

gggagccaat tcacaattca ctaagtgact aaagtaagtt aaacttgtgt agactaagca 60

tgtaattttt aagttttatt ttaatgaatt aaaatatttg ttaaccaact ttaaagtcag 120

tcctgtgtat acctagatat tagtcagttg gtgccagata gaagacaggt tgtgttttta 180

tcctgtggct tgtgtagtgt cctgggattc tctgcccccct ctgagtagag tgttgtggga 240

taaaggaatc tctcaggg 258

<210> 10
<211> 549
<212> DNA
<213> Artificial Sequence

<220>
<223> Description of Artificial Sequence: Designed fragment based on UCK2

<400> 10

cccaacggcg gcgagccctt ccttataggc gtcagcgggg gaacagctag cggcaagtct 60

tccgtgtgtg ctaagatcgt gcagctcctg gggcagaatg aggtggacta tcgccagaag 120

caggtggtca tcctgagcca ggatagcttc taccgtgtcc ttacctcgga gcagaaggcc 180

aaagccctga agggccagtt caactttgac cacccggatg cctttgacaa tgaactcatt 240

ctcaaaacac tcaaagaaat cactgaaggg aaaacagtcc agatccccgt gtatgacttt 300

gtctcccatt cccggaagga ggagacagtt actgtctatc ccgcagacgt ggtgctcttt 360

gaagggatcc tggccttcta ctcccaggag gtacgagacc tgttccagat gaagcttttt 420

gtggatacag atgcggacac ccggctctca cgcagagtat taagggacat cagcgagaga 480

ggcagggatc ttgagcagat tttatctcag tacattacgt tcgtcaagcc tgcctttgag 540

gaattctgc 549

<210> 11
<211> 384
<212> DNA
<213> Artificial Sequence

<220>
<223> Description of Artificial Sequence: Designed fragment based on UP

<400> 11

ttctgcagag ctgagcgagt tcaccacagt ggtggggaac accatgtgca ccttggactt 60

ctatgaaggg caaggccgtc tggatggggc tctctgctcc tacacggaga aggacaagca 120

ggcgtatctg gaggcagcct atgcagccgg cgtccgcaat atcgagatgg agtcctcggt 180

gtttgccgcc atgtgcagcg cctgcggcct ccaagcggcc gtggtgtgtg tcaccctcct 240

gaaccgcctg gaaggggacc agatcagcag ccctcgcaat gtgctcagcg agtaccagca 300

gaggccgcag cggctggtga gctacttcat caagaagaaa ctgagcaagg cctgagcgct 360

gccctgcacc tccgcagacc tgct 384

<210> 12
<211> 302
<212> DNA
<213> Artificial Sequence

<220>

<223> Description of Artificial Sequence: Designed fragment based on CDA

<400> 12

ggcatctgtg ctgaacggac cgctatccag aaggccgtct cagaagggta caaggatttc 60

agggcaattg ctatcgccag tgacatgcaa gatgatttta tctctccatg tggggcctgc 120

aggcaagtca tgagagagtt tggcaccaac tggcccgtgt acatgaccaa gccggatggt 180

acgtatattg tcatgacggt ccaggagctg ctgccctcct cctttgggcc tgaggacctg 240

cagaagactc agtgacagcc agagaatgcc cactgcctgt aacagccacc tggagaactt 300

ca 302

<210> 13
<211> 274
<212> DNA
<213> Artificial Sequence

<220>
<223> Description of Artificial Sequence: Designed fragment based on NT5

<400> 13

ttctaaacct gcacttgtcc ctctccagca agaggctagc actgaattca ttctactcat 60

actacacacc cagttatgga atgtccagag ttctcgaaga aaataaatga ctttaggaag 120

aggtatacat tttttaagtc gctctgcctc caaatctgaa cagtcactgt aaatcattct 180

taagcccaga tatgagaact tctgctggaa agtgggaccc tctgagtggg tggtcagaaa 240

atacccatgc tgatgaaatg acctatgccc aaag 274

<210> 14
<211> 254
<212> DNA
<213> Artificial Sequence

<220>
<223> Description of Artificial Sequence: Designed fragment based on IMPD

<400> 14

tcgtgcccta cctcatagca ggcatccaac acggctgcca ggatatcggg gcccgcagcc 60

tgtctgtcct tcggtccatg atgtactcag gagagctcaa gtttgagaag cggaccatgt 120

cgccccagat tgagggtggt gtccatggcc tgcactctta cgaaaagcgg ctgtactgag 180

gacagcggtg gaggccgagg tggtggaggg gatgcacccc agtgtccact tttgggcaca 240

ggctccctcc ataa 254

<210> 15
<211> 304
<212> DNA
<213> Artificial Sequence

<220>
<223> Description of Artificial Sequence: Designed fragment based on MTHFD1

<400> 15

tcctggctct caccacttct ctagaagaca tgagagagag actgggcaaa atggtggtgg 60

catccagtaa gaaaggagag cccgtcagtg ccgaagatct gggggtgagt ggtgcactga 120

cagtgcttat gaaggacgca atcaagccca atctcatgca gacactggag ggcactccag 180

tgtttgtcca tgctggcccg tttgccaaca tcgcacatgg caattcctcc atcattgcag 240

accagatcgc actcaagctt gttggcccag aagggtttgt agtgacggaa gcaggatttg 300

gagc 304

<210> 16
<211> 331
<212> DNA
<213> Artificial Sequence

<220>
<223> Description of Artificial Sequence: Designed fragment based on RP2

<400> 16

aagagtggac ttctcggccc gtactgtcat cacccccgac cccaacctct ccattgacca 60

ggttggcgtg ccccgctcca ttgctgccaa catgaccttt gcggagattg tcacccccctt 120

caacattgac agacttcaag aactagtgcg cagggggaac agtcagtacc caggcgccaa 180

gtacatcatc cgagacaatg gtgatcgcat tgacttgcgt ttccacccca agcccagtga 240

ccttcacctg cagaccggct ataaggtgga acggcacatg tgtgatgggg acattgttat 300

cttcaaccgg cagccaactc tgcacaaaat g 331

<210> 17
<211> 285
<212> DNA
<213> Artificial Sequence

<220>
<223> Description of Artificial Sequence: Designed fragment based on UMPK

<400> 17

tctaaacctg aaagcatcct tgaaatcatg cttgaatatt gctttgatag ctgctatcat 60

gacccctttt taaggcaatt ctaatctttc ataactacat ctcaattagt ggctggaaag 120

tacatggtaa aacaaagtaa attttttat gttcttttt tggtcacagg agtagacagt 180

gaattcaggt ttaacttcac cttagttatg gtgctcacca aacgaagggt atcagctatt 240

tttttttaaa ttcaaaaaga atatcccttt tatagtttgt gcctt 285

<210> 18
<211> 265
<212> DNA
<213> Artificial Sequence

<220>
<223> Description of Artificial Sequence: Designed fragment based on CTPS

<400> 18

ccatcggtca ccttgtttct caactacctc gcatcattgc agatcgtagc gcgttgcctg 60

tcgctttccc ttggatacct agaccgttat aaagtgtgcc acatggactt accgagcatg 120

gagagaggat tttagctagg atttgaacac ttggtgctgg gaacctcagg gtattgcttg 180

ccactaagcc atgaaaccag agacaaaatc tctatactgc cctgagttgg ggggaattct 240

cagtgccaac tgtggctggt cctca 265

<210> 19
<211> 368
<212> DNA
<213> Artificial Sequence

<220>
<223> Description of Artificial Sequence: Designed fragment based on DCD

<400> 19

cagggtggtg gcacattatc cctctggggg gtggggacgc ctgttgtttt ggctcaattt 60

gggtttgttg gtcacatgga gctcttccat ttcgtttagc tgaataatga gttgttccta 120

gaggagacag cctgtctctc cttgttgccc ccaaagccca tgccctgccg tggtggcagc 180

tggggctgtg gatgggaggg gtccccaaca tggatgtgtt gcccctcctc cgcatgccaa 240

cgcagttcat gtacaaggcc cctctgcaac tggagagaaa attaattcct atcccgtgag 300

tggattgtga gaaattccac ccacgtggag acagcttact gcagcactgt tggtgttcgg 360

agctcttc 368

<210> 20
<211> 422
<212> DNA
<213> Artificial Sequence

<220>
<223> Description of Artificial Sequence: Designed fragment based on DCK

<400> 20

gatctgtgta tagtgacagg tatatttttg catctaattt gtatgaatct gaatgcatga 60
atgagacaga gtggacaatt tatcaagact ggcatgactg gatgaataac caatttggcc 120
aaagccttga attggatgga atcatttatc ttcaagccac tccagagaca tgcttacata 180
gaatatattt acggggaaga aatgaagagc aaggcattcc tcttgaatat ttagagaagc 240
ttcattataa acatgaaagc tggctcctgc ataggacact gaaaaccaac ttcgattatc 300
ttcaagaggt gcctatctta acactggatg ttaatgaaga ctttaaagac aaatatgaaa 360
gtctggttga aaaggtcaaa gagtttttga gtactttgtg atcttgctga agactacagg 420
ca 422

<210> 21
<211> 322
<212> DNA
<213> Artificial Sequence

<220>
<223> Description of Artificial Sequence: Designed fragment based on PRPS

<400> 21

attcagcaga agacccggct tgctccagtg tagctttcta catcccacat caggtatatt 60
agagcttatc cgaactgggg aaagacggat tgagattaac tgctgggacc tcctacctgc 120
attatctcat tctggcttcc ttgataattc tgtgggcctt gcagctttaa ctatagctca 180
gctgctgcaa gatttcagac ttttgaggat gttgtgtgag ggtgtttgac tgtgactggg 240
gaagctcaga ctactttgta tgtgaatgct tcagggtttt ctttgttgag aacaactagc 300
aacaaaggca acccatgtgt ga 322

<210> 22
<211> 391
<212> DNA
<213> Artificial Sequence

<220>
<223> Description of Artificial Sequence: Designed fragment based on HPRT1

<400> 22

gccatctgct tagtagagct ttttgcatgt atcttctaag aattttatct gttttgtact 60

ttagaaatgt cagttgctgc attcctaaac tgtttatttg cactatgagc ctatagacta 120

tcagttccct ttgggcggat tgttgtttaa cttgtaaatg aaaaaattct cttaaaccac 180

agcactattg agtgaaacat tgaactcata tctgtaagaa ataaagagaa gatatattag 240

ttttttaatt ggtattttaa tttttatata tgcaggaaag aatagaagtg attgaatatt 300

gttaattata ccaccgtgtg ttagaaaagt aagaagcagt caattttcac atcaaagaca 360

gcatctaaga agttttgttc tgtcctggaa t 391

<210> 23
<211> 220
<212> DNA
<213> Artificial Sequence

<220>
<223> Description of Artificial Sequence: Designed fragment based on FPGS

<400> 23

cgcatgctca ataccctgca gaccaatgcc ggctacctgg agcaggtgaa gcgccagcgg 60

ggtgaccctc agacacagtt ggaagccatg gaactgtacc tggcacggag tgggctgcag 120

gtggaggact tggaccggct gaacatcatc cacgtcactg ggacgaaggg gaagggctcc 180

acctgtgcct tcacggaatg tatcctccga agctatggcc 220

<210> 24
<211> 364
<212> DNA
<213> Artificial Sequence

<220>
<223> Description of Artificial Sequence: Designed fragment based on NDKA

<400> 24

cggtagttgc catggtctgg gaggggctga atgtggtgaa gacgggccga gtcatgctcg 60

gggagaccaa ccctgcagac tccaagcctg ggaccatccg tggagacttc tgcatacaag 120

ttggcaggaa cattatacat ggcagtgatt ctgtggagag tgcagagaag gagatcggct 180

tgtggtttca ccctgaggaa ctggtagatt acacgagctg tgctcagaac tggatctatg 240

aatgacagga gggcagacca cattgctttt cacatccatt tcccctcctt cccatgggca 300

gaggaccagg ctgtaggaaa tctagttatt tacaggaact tcatcataat ttggagggaa 360

gctc 364

<210> 25
<211> 372
<212> DNA
<213> Artificial Sequence

<220>
<223> Description of Artificial Sequence: Designed fragment based on NDKB

<400> 25

acctgaaaga ccgaccattc ttccctgggc tggtgaagta catgaactca gggccggttg 60

tggccatggt ctgggagggg ctgaacgtgg tgaagacagg ccgagtgatg cttggggaga 120

ccaatccagc agattcaaag ccaggcacca ttcgtgggga cttctgcatt caggttggca 180

ggaacatcat tcatggcagt gattcagtaa aaagtgctga aaaagaaatc agcctatggt 240

ttaagcctga agaactggtt gactacaagt cttgtgctca tgactgggtc tatgaataag 300

aggtggacac aacagcagtc tccttcagca cggcgtggtg tgtccctgga cacagctctt 360

cattccattg ac 372

<210> 26
<211> 406
<212> DNA
<213> Artificial Sequence

<220>
<223> Description of Artificial Sequence: Designed fragment based on APRT

<400> 26

```
acatctcgcc cgtcctgaag gaccccgcct ccttccgcgc cgccatcggc ctcctggcgc 60
gacacctgaa ggcgacccac gggggccgca tcgactacat cgcaggccta gactcccgag 120
gcttcctctt tggcccctcc ctggcccagg agcttggact gggctgcgtg ctcatccgaa 180
agcgggggaa gctgccaggc cccactctgt gggcctccta ttccctggag tacgggaagg 240
ctgagctgga gattcagaaa gacgccctgg agccaggaca gagggtggtc gtcgtggatg 300
atctgctggc cactggtgga accatgaacg ctgcctgtga gctgctgggc cgcctgcagg 360
ctgaggtcct ggagtgcgtg agcctggtgg agctgacctc gcttaa                406
```

<210> 27
<211> 369
<212> DNA
<213> Artificial Sequence

<220>
<223> Description of Artificial Sequence: Designed fragment based on AK

<400> 27

```
ctgcaccgtt tattagccag ttctacaagg aatcattgat gaaagttatg ccttatgttg 60
atatactttt tggaaatgag acagaagctg ccacttttgc tagagagcaa ggctttgaga 120
ctaaagacat taaagagata gccaaaaaga cacaagccct gccaaagatg aactcaaaga 180
ggcagcgaat cgtgatcttc acccaaggga gagatgacac tataatggct acagaaagtg 240
aagtcactgc ttttgctgtc ttggatcaag accagagaga aattattgat accaatggag 300
ctggagatgc atttgttgga ggttttctgt ctcaactggt ctctgacaag ccactgactg 360
aatgtatcc                                                         369
```

<210> 28
<211> 317
<212> DNA
<213> Artificial Sequence

<220>
<223> Description of Artificial Sequence: Designed fragment based on ERCC1

<400> 28

```
tgatacccct cgacgaggat gaggtccctc ctggagtggc caagccctta ttccgatcta 60
cacagagcct tcccactgtg gacacctcgg cccaggcggc ccctcagacc tacgccgaat 120
atgccatctc acagcctctg gaaggggctg gggccacgtg ccccacaggg tcagagcccc 180
tggcaggaga gacgcccaac caggccctga aacccggggc aaaatccaac agcatcattg 240
tgagccctcg gcagaggggc aatcccgtac tgaagttcgt gcgcaacgtg ccctgggaat 300
ttggcgacgt aattccc                                                317
```

<210> 29
<211> 391
<212> DNA
<213> Artificial Sequence

<220>
<223> Description of Artificial Sequence: Designed fragment based on UDG

<400> 29

```
atctggccca gaaattaggg ctcaatttcc tgattgtagt agaggttaag attgctgtga 60
gctttatcag ataagagacc gagagaagta agctgggtct tgttattcct tgggtgttgg 120
tggaataagc agtggaattt gaacaaggaa gaggagaaaa gggaattttg tctttatggg 180
gtggggtgat tttctcctag ggttatgtcc agttggggtt tttaaggcag cacagactgc 240
caagtactgt ttttttaac cgactgaaat cactttggga tattttttcc tgcaacactg 300
gaaagtttta gtttttaag aagtactcat gcagatatat atatatatat ttttcccagt 360
ccttttttta agagacggtc tttattgggt c                                391
```

<210> 30
<211> 410
<212> DNA
<213> Artificial Sequence

<220>
<223> Description of Artificial Sequence: Designed fragment based on PARP

<400> 30

aaggcgaatg ccagcgttac aagcccttta agcagcttca taaccgaaga ttgctgtggc 60
acgggtccag gaccaccaac tttgctggga tcctgtccca gggtcttcgg atagccccgc 120
ctgaagcgcc cgtgacaggc tacatgtttg gtaaagggat ctatttcgct gacatggtct 180
ccaagagtgc caactactac catacgtctc agggagaccc aataggctta atcctgttgg 240
gagaagttgc ccttggaaac atgtatgaac tgaagcacgc ttcacatatc agcaggttac 300
ccaagggcaa gcacagtgtc aaaggtttgg gcaaaactac ccctgatcct tcagctaaca 360
ttagtctgga tggtgtagac gttcctcttg ggaccgggat ttcatctggt 410

<210> 31
<211> 309
<212> DNA
<213> Artificial Sequence

<220>
<223> Description of Artificial Sequence: Designed fragment based on LIG1

<400> 31

actggcagtg cttccacagc caagaagata gacatcatca aaggcctctt tgtggcctgc 60
cgccactcag aagcccggtt catcgctagg tccctgagcg gacggctgcg ccttgggctg 120
gcagagcagt cggtgctggc tgccctctcc caggcagtga gcctcacgcc cccgggccaa 180
gaattcccac cagccatggt ggatgctggg aagggcaaga cagcagaggc cagaaagacg 240
tggctggagg agcaaggcat gatcctgaag cagacgttct gcgaggttcc cgacctggac 300
cgaattatc 309

<210> 32
<211> 355
<212> DNA
<213> Artificial Sequence

<220>
<223> Description of Artificial Sequence: Designed fragment based on LIG3

<400> 32

caaggtggcc cactttaagg actacattcc ccaggctttt cctgggggcc acagcatgat 60
cttggattct gaagtgcttc tgattgacaa caagacaggc aaaccactgc cctttgggac 120
tctgggagta cacaagaaag cagccttcca ggatgctaat gtctgcctgt ttgtttttga 180
ttgtatctac tttaatgatg tcagcttgat ggacagacct ctgtgtgagc ggcggaagtt 240
tcttcatgac aacatggttg aaattccaaa ccggatcatg ttctcagaaa tgaagcgagt 300
cacaaaagct ttggacttgg ctgacatgat aacccgggtg atccaggagg gattg 355

<210> 33
<211> 411
<212> DNA
<213> Artificial Sequence

<220>
<223> Description of Artificial Sequence: Designed fragment based on LIG4

<400> 33

ggcatctggt aagctcgcat ctaaacacct ttatataggt ggtgatgatg aaccacaaga 60
aaaaaagcgg aaagctgccc caaagatgaa gaaagttatt ggaattattg agcacttaaa 120
agcacctaac cttactaacg ttaacaaaat ttctaatata tttgaagatg tagagttttg 180
tgttatgagt ggaacagata gccagccaaa gcctgacctg gagaacagaa ttgcagaatt 240
tggtggttat atagtacaaa atccaggccc agacacgtac tgtgtaattg cagggtctga 300
gaacatcaga gtgaaaaaca taattttgtc aaataaacat gatgttgtca agcctgcatg 360
gcttttagaa tgttttaaga ccaaaagctt tgtaccatgg cagcctcgct t 411

<210> 34
<211> 335
<212> DNA
<213> Artificial Sequence

<220>
<223> Description of Artificial Sequence: Designed fragment based on POLB

<400> 34

cgccatgagc aaacggaagg cgccgcagga gactctcaac gggggaatca ccgacatgct 60

cacagaactc gcaaactttg agaagaacgt gagccaagct atccacaagt acaatgctta 120

cagaaaagca gcatctgtta tagcaaaata cccacacaaa ataaagagtg gagctgaagc 180

taagaaattg cctggagtag gaacaaaaat tgctgaaaag attgatgagt ttttagcaac 240

tggaaaatta cgtaaactgg aaaagattcg gcaggatgat acgagttcat ccatcaattt 300

cctgactcga gttagtggca ttggtccatc tgctg 335

<210> 35
<211> 262
<212> DNA
<213> Artificial Sequence

<220>
<223> Description of Artificial Sequence: Designed fragment based on POLD

<400> 35

ccaccttcat ccgtatcatg gaccccgacg tgatcaccgg ttacaacatc cagaacttcg 60

accttccgta cctcatctct cgggcccaga ccctcaaggt acaaacattc cctttcctgg 120

gccgtgtggc cggcctttgc tccaacatcc gggactcttc attccagtcc aagcagacgg 180

gccggcggga caccaaggtt gtcagcatgg tgggccgcgt gcagatggac atgctgcagg 240

tgctgctgcg ggagtacaag ct 262

<210> 36
<211> 368
<212> DNA
<213> Artificial Sequence

<220>
<223> Description of Artificial Sequence: Designed fragment based on XRCC1

<400> 36

ctgtcgccat ctgttcccaa gagacctaaa ttgccagctc caactcgtac cccagccaca 60

gccccagtcc ctgcccgagc acagggggca gtgacaggca aaccccgagg agaaggcacc 120

gagcccagac gaccccgagc tggcccagag gagctgggga agatccttca gggtgtggta 180

gtggtgctga gtggcttcca gaaccccttc cgctccgagc tgcgagataa ggccctagag 240

cttggggcca agtatcggcc agactggacc cgggacagca cgcacctcat ctgtgccttt 300

gccaacaccc ccaagtacag ccaggtccta ggcctgggag gccgcatcgt gcgtaaggag 360

tgggtgct 368

<210> 37
<211> 324
<212> DNA
<213> Artificial Sequence

<220>
<223> Description of Artificial Sequence: Designed fragment based on TOP1

<400> 37

tatttcaaag cccagacgga agctcggaaa cagatgagca aggaagagaa actgaaaatc 60

aaagaggaga atgaaaaatt actgaaagaa tatggattct gtattatgga taaccacaaa 120

gagaggattg ctaacttcaa gatagagcct cctggacttt tccgtggccg cggcaaccac 180

cccaagatgg gcatgctgaa gagacgaatc atgcccgagg atataatcat caactgtagc 240

aaagatgcca aggttccttc tcctcctcca ggacataagt ggaaagaagt ccggcatgat 300

aacaaggtta cttggctggt ttcc 324

<210> 38
<211> 265
<212> DNA
<213> Artificial Sequence

<220>
<223> Description of Artificial Sequence: Designed fragment based on MDR1

<400> 38

tcagttaccc atctcgaaaa gaagttaaga tcttgaaggg tctgaacctg aaggtgcaga 60

gtgggcagac ggtggccctg gttggaaaca gtggctgtgg gaagagcaca acagtccagc 120

tgatgcagag gctctatgac cccacagagg ggatggtcag tgttgatgga caggatatta 180

ggaccataaa tgtaaggttt ctacgggaaa tcattggtgt ggtgagtcag gaacctgtat 240

tgtttgccac cacgatagct gaaaa 265

<210> 39
<211> 256
<212> DNA
<213> Artificial Sequence

<220>
<223> Description of Artificial Sequence: Designed fragment based on ENT1

<400> 39

tcagccaggg aaaaccgaga acaccatcac catgacaacc agtcaccagc ctcaggacag 60

atacaaagct gtctggctta tcttcttcat gctgggtctg ggaacgctgc tcccgtggaa 120

ttttttcatg acggccactc agtatttcac aaaccgcctg gacatgtccc agaatgtgtc 180

cttggtcact gctgaactga gcaaggacgc ccaggcgtca gccgcccctg cagcaccctt 240

gcctgagcgg aactct 256

<210> 40
<211> 399
<212> DNA
<213> Artificial Sequence

<220>
<223> Description of Artificial Sequence: Designed fragment based on MRP1

<400> 40

```
cctgtttgcg gtgatctcca ggcacagcct cagtgctggc ttggtgggcc tctcagtgtc 60
ttactcattg caggtcacca cgtacttgaa ctggctggtt cggatgtcat ctgaaatgga 120
aaccaacatc gtggccgtgg agaggctcaa ggagtattca gagactgaga aggaggcgcc 180
ctggcaaatc caggagacag ctccgcccag cagctggccc caggtgggcc gagtggaatt 240
ccggaactac tgcctgcgct accgagagga cctggacttc gttctcaggc acatcaatgt 300
cacgatcaat gggggagaaa aggtcggcat cgtggggcgg acgggagctg ggaagtcgtc 360
cctgaccctg ggcttatttc ggatcaacga gtctgccga                        399
```

<210> 41
<211> 353
<212> DNA
<213> Artificial Sequence

<220>
<223> Description of Artificial Sequence: Designed fragment based on TOP2A

<400> 41

```
gtgctttttg accacgtagg ctgtttaaag aaatatgaca cggtgttgga tattctaaga 60
gacttttttg aactcagact taaatattat ggattaagaa aagaatggct cctaggaatg 120
cttggtgctg aatctgctaa actgaataat caggctcgct ttatcttaga gaaaatagat 180
ggcaaaataa tcattgaaaa taagcctaag aaagaattaa ttaaagttct gattcagagg 240
ggatatgatt cggatcctgt gaaggcctgg aaagaagccc agcaaaaggt tccagatgaa 300
gaagaaaatg aagagagtga caacgaaaag gaaactgaaa agagtgactc cgt        353
```

<210> 42
<211> 374
<212> DNA
<213> Artificial Sequence

<220>
<223> Description of Artificial Sequence: Designed fragment based on TOP2B

<400> 42

ttacgtaagg agtggcttgt gggaatgttg ggagcagaat ctacaaagct taacaatcaa 60

gcccgtttca ttttagagaa gatacaaggg aaaattacta tagagaatag gtcaaagaaa 120

gatttgattc aaatgttagt ccagagaggt tatgaatctg acccagtgaa agcctggaaa 180

gaagcacaag aaaaggcagc agaagaggat gaaacacaaa accagcatga tgatagttcc 240

tccgattcag gaactccttc aggcccagat tttaattata ttttaaatat gtctctgtgg 300

tctcttacta aagaaaaagt tgaagaactg attaaacaga gagatgcaaa agggcgagag 360

gtcaatgatc ttaa 374

<210> 43
<211> 212
<212> DNA
<213> Artificial Sequence

<220>
<223> Description of Artificial Sequence: Designed fragment based on Hsp27

<400> 43

ctacagccgc gcgctcagcc ggcaactcag cagcggggtc tcggagatcc ggcacactgc 60

ggaccgctgg cgcgtgtccc tggatgtcaa ccacttcgcc ccggacgagc tgacggtcaa 120

gaccaaggat ggcgtggtgg agatcaccgg caagcacgag gagcggcagg acgagcatgg 180

ctacatctcc cggtgcttca cgcggaaata ca 212

<210> 44
<211> 323
<212> DNA
<213> Artificial Sequence

<220>
<223> Description of Artificial Sequence: Designed fragment based on ENT2

<400> 44

```
tgaccagagg gttcagagtg ggaggcaggg ccagcccagg ccaggagcgc ctcatcttcc 60
caggcctcag ccacccaggg taaaaggtgc cagggaagtt gtgggcacct gagaggagga 120
acagatgtgg aggacctgag ggtgctcaaa gggccaggct cagcctcaag cagtgttttc 180
attgccaaca cttactgtac ccactccgca gagccccgct gggcctgggc cccagggcca 240
cagctagcct gcatgtgtgt actgcacttt acagtttgca aagctcttcc atacccactc 300
tctcaccgaa gcctaattga ggc                                         323
```

<210> 45
<211> 296
<212> DNA
<213> Artificial Sequence

<220>
<223> Description of Artificial Sequence: Designed fragment based on E2F1

<400> 45

```
cgatgttttc ctgtgccctg aggagaccgt aggtgggatc agccctggga agaccccatc 60
ccaggaggtc acttctgagg aggagaacag ggccactgac tctgccacca tagtgtcacc 120
accaccatca tctcccccct catccctcac cacagatccc agccagtctc tactcagcct 180
ggagcaagaa ccgctgttgt cccggatggg cagcctgcgg gctcccgtgg acgaggaccg 240
cctgtccccg ctggtggcgg ccgactcgct cctggagcat gtgcgggagg acttct     296
```

<210> 46
<211> 335
<212> DNA
<213> Artificial Sequence

<220>
<223> Description of Artificial Sequence: Designed fragment based on p53

<400> 46

gctcagatag cgatggtctg gcccctcctc agcatcttat ccgagtggaa ggaaatttgc 60

gtgtggagta tttggatgac agaaacactt ttcgacatag tgtggtggtg ccctatgagc 120

cgcctgaggt tggctctgac tgtaccacca tccactacaa ctacatgtgt aacagttcct 180

gcatgggcgg catgaaccgg aggcccatcc tcaccatcat cacactggaa gactccagtg 240

gtaatctact gggacggaac agctttgagg tgcgtgtttg tgcctgtcct gggagagacc 300

ggcgcacaga ggaagagaat ctccgcaaga aaggg 335

<210> 47
<211> 279
<212> DNA
<213> Artificial Sequence

<220>
<223> Description of Artificial Sequence: Designed fragment based on VEGFB

<400> 47

cagaggaaag tggtgtcatg gatagatgtg tatactcgcg ctacctgcca gccccgggag 60

gtggtggtgc ccttgactgt ggagctcatg ggcaccgtgg ccaaacagct ggtgcccagc 120

tgcgtgactg tgcagcgctg tggtggctgc tgccctgacg atggcctgga gtgtgtgccc 180

actgggcagc accaagtccg gatgcagatc ctcatgatcc ggtacccgag cagtcagctg 240

ggggagatgt ccctggaaga acacagccag tgtgaatgc 279

<210> 48
<211> 368
<212> DNA
<213> Artificial Sequence

<220>
<223> Description of Artificial Sequence: Designed fragment based on ITGA3

<400> 48

gctgtatccc acggagatca ccgtccatgg caatgggtcc tggccctgcc gaccacctgg 60

agaccttatc aaccctctca acctcactct ttctgaccct ggggacaggc catcatcccc 120

acagcgcagg cgccgacagc tggatccagg gggaggccag ggccccccac ctgtcactct 180

ggctgctgcc aaaaaagcca agtctgagac tgtgctgacc tgtgccacag ggcgtgccca 240

ctgtgtgtgg ctagagtgcc ccatccctga tgcccccgtt gtcaccaacg tgactgtgaa 300

ggcacgagtg tggaacagca ccttcatcga ggattacaga gactttgacc gagtccgggt 360

aaatggct 368

<210> 49
<211> 270
<212> DNA
<213> Artificial Sequence

<220>
<223> Description of Artificial Sequence: Designed fragment based on S0D2

<400> 49

atctgggctc caggcagaag cacagcctcc ccgacctgcc ctacgactac ggcgccctgg 60

aacctcacat caacgcgcag atcatgcagc tgcaccacag caagcaccac gcggcctacg 120

tgaacaacct gaacgtcacc gaggagaagt accaggaggc gttggccaag ggagatgtta 180

cagcccagat agctcttcag cctgcactga agttcaatgg tggtggtcat atcaatcata 240

gcattttctg gacaaacctc agccctaacg 270

<210> 50
<211> 260
<212> DNA
<213> Artificial Sequence

<220>
<223> Description of Artificial Sequence: Designed fragment based on S0D1

<400> 50

gcctagcgag ttatggcgac gaaggccgtg tgcgtgctga agggcgacgg cccagtgcag 60

ggcatcatca atttcgagca gaaggaaagt aatggaccag tgaaggtgtg gggaagcatt 120

aaaggactga ctgaaggcct gcatggattc catgttcatg agtttggaga taatacagca 180

ggctgtacca gtgcaggtcc tcactttaat cctctatcca gaaaacacgg tgggccaaag 240

gatgaagaga ggcatgttgg 260

<210> 51
<211> 316
<212> DNA
<213> Artificial Sequence

<220>
<223> Description of Artificial Sequence: Designed fragment based on PCNA

<400> 51

actccgccac catgttcgag gcgcgcctgg tccagggctc catcctcaag aaggtgttgg 60

aggcactcaa ggacctcatc aacgaggcct gctgggatat tagctccagc ggtgtaaacc 120

tgcagagcat ggactcgtcc cacgtctctt tggtgcagct caccctgcgg tctgagggct 180

tcgacaccta ccgctgcgac cgcaacctgg ccatgggcgt gaacctcacc agtatgtcca 240

aaatactaaa atgcgccggc aatgaagata tcattacact aagggccgaa gataacgcgg 300

ataccttggc gctagt 316

<210> 52
<211> 333
<212> DNA
<213> Artificial Sequence

<220>
<223> Description of Artificial Sequence: Designed fragment based on GAPDH

<400> 52

cgtcatgggt gtgaaccatg agaagtatga caacagcctc aagatcatca gcaatgcctc 60

ctgcaccacc aactgcttag cacccctggc caaggtcatc catgacaact ttggtatcgt 120

ggaaggactc atgaccacag tccatgccat cactgccacc cagaagactg tggatggccc 180

ctccgggaaa ctgtggcgtg atggccgcgg ggctctccag aacatcatcc ctgcctctac 240

tggcgctgcc aaggctgtgg gcaaggtcat ccctgagcta gacgggaagc tcactggcat 300

ggccttccgt gtccccactg ccaacgtgtc agt 333

<210> 53
<211> 264
<212> DNA
<213> Artificial Sequence

<220>
<223> Description of Artificial Sequence: Designed fragment based on ACTB

<400> 53

cattggcaat gagcggttcc gctgccctga ggcactcttc cagccttcct tcctgggcat 60

ggagtcctgt ggcatccacg aaactacctt caactccatc atgaagtgtg acgtggacat 120

ccgcaaagac ctgtacgcca acacagtgct gtctggcggc accaccatgt accctggcat 180

tgccgacagg atgcagaagg agatcactgc cctggcaccc agcacaatga agatcaagat 240

cattgctcct cctgagcgca agta 264

<210> 54
<211> 361
<212> DNA
<213> Artificial Sequence

<220>
<223> Description of Artificial Sequence: Designed fragment based on RSP9

<400> 54

tcctgggcct gaagatagag gatttcttag agagacgcct gcagacccag gtcttcaagc 60

tgggcttggc caagtccatc caccacgctc gcgtgctgat ccgccagcgc catatcaggg 120

tccgcaagca ggtggtgaac atcccgtcct tcattgtccg cctggattcc cagaagcaca 180

ttgacttctc tctgcgctct cctacggggg ttggccgccc gggccgcgtg aagaggaaga 240

atgccaagaa gggccagggt ggggctgggg ctggagacga cgaggaggag gattaagtcc 300

acctgtccct cctgggctgc tggattgtct cgttttcctg ccaaataaac aggatcagcg 360

c 361

## Description

Technical Field

The present invention relates to a DNA array for measuring sensitivity to an anticancer agent, and to a method for measuring such sensitivity by use of the DNA array.

Background Art

Generally speaking, anticancer agents are not necessarily satisfactory in terms of therapeutic effects on cancer patients, permitting relatively high incidences of adverse side effects. In recent years, demand has arisen for proper use of anticancer agents and treatment that is custom-tailored to the conditions of the individual patient, wherein before administration of an anticancer agent to a patient, a tissue specimen or a similar specimen is collected from the patient, and while the specimen is used as a test sample, the expression level of a gene associated with sensitivity to the anticancer agent is measured. This allows proper selection of patients who would be expected to exhibit satisfactory effect of the drug or a low level of adverse side effect.

Clinical research efforts have been devoted toward establishing criteria for proper use of 5-fluorouracil (5-FU) agents and other antimetabolites among popular anticancer agents. Typical target genes for serving assay are genes of thymidylate synthase (EC2.1.1.45, hereinafter referred to as "TS"), dihydropyrimidine dehydrogenase (EC1.3.1.2, hereinafter referred to as "DPD"), and thymidine phosphorylase (EC2.4.2.4, hereinafter referred to as "TP"), which are considered to be associated with sensitivity to this antimetabolite. Expression patterns of these genes or enzymes are analyzed through biochemical assays for determining enzymatic activity (Clinical Cancer Research, 5, 883-889, 1999), immunoassays using antibodies (Cancer and Chemotherapy, 24(6): 705-712, 1997), or mRNA assays making use of Northern hybridization or RT-PCR (Clinical Cancer Research, 6, 1322-1327, 2000), and the results therefrom are used as indices of proper use. In cancer chemotherapy using cisplatin, DNA repair enzymes such as ERCC1 are suggested to be useful as factors that predict the effect of therapy (Journal of Clinical Oncology, 16, 309-316, 1998).

Meanwhile, the DNA microarray method has recently come into widely use, as it enables simultaneous analysis of mRNA expression of several hundred to some dozens of thousands of diversified genes. This technique is suitable for comprehensive analysis of genes, and thus is expected to be applied to realization of proper use of anticancer agents in the future.

In the human body, 5-fluorouracil and other antimetabolites are metabolized through a variety of processes. Therefore, only the gene expression analysis of the above-mentioned 3 types of genes (TS, DPD, and TP) is not sufficient for predicting sensitivity to antimetabolites. If gene expression can be analyzed in broader ranges, sensitivity would be better predicted. In actual settings of cancer chemotherapies, combination therapy is commonly performed, in which several types of anticancer agents are used in combination. In such cases, if expression of genes that are associated with sensitivity to each drug can be analyzed comprehensively, suitable combination chemotherapy can be designed for each individual patient.

Biochemical assays for measuring enzyme activity, immunoassays making use of antibodies, Northern hybridization, and RT-PCR mRNA assays provide quantitatively precise data. However, in principle, they require one assay for each gene, and therefore, they are not suitable for simultaneous analysis of diversified modes of gene expression.

WO 01/32 928 describes methods of determining individual hyper-sensitivity to an agent and DNA arrays therefor.

In contrast, the DNA microarray method is useful for the analysis of diversified gene expressions. However, this method is far inferior to Northern hybridization or RT-PCR in terms of quantitative preciseness. One of the main reasons may be explained as follows: Northern hybridization and RT-PCR can designate, based on the size of RNA or PCR product, the specificity to a specific gene of interest, whereas the DNA microarray method cannot, because this method relies on dot blots which do not show the molecular size, leading to false detection; i.e., detection of non-specific signals attributed to expression of genes other than the gene of interest (so-called cross-hybridization). Specificity to a gene of interest is affected by the selection of DNA fragments to be arrayed (hereinafter such DNA fragments will be referred to as the target fragments); i.e., selection of portions (regions) of the gene (full-length cDNA) as target fragments. Specificity obtained in an ordinarily performed DNA microarray method has not yet been substantiated, although the target fragments employed in the method are determined among the regions that have been predicted to have high specificity from computation (regions showing minor nucleotide sequence overlapping with corresponding sequences of other genes which are publicly available from databases) by use of design assistance software. Moreover, primers which have specificity to each individual gene employed in PCR, which is performed for preparing target fragments through amplification, may migrate to a target fragment solution and be included in arrays, raising the risk of causing non-uniform background. From these factors, even for a single specimen (a sample derived form a living organism), expression level of a specific gene analyzed through the DNA microarray method often disagrees with the results obtained through Northern hybridization or RT-PCR, and therefore, it is improper to conclude existence of a difference in gene expression solely from the results obtained from the DNA microarray method. According to a generally employed test procedure, genes which are likely to show different levels of expression are roughly identified through a first screening by use of the DNA microarray method, and then, through a second screening, like Northern hybridization or RT-PCR is performed to verify that differences in expression level in fact exist. Thus, in such a scheme, at least 2 assay methods must be employed.

Accordingly, an object of the present invention is to provide means for measuring, in single measurement, sensitivity to an antimetabolite-type anticancer agent or to a combination of such an anticancer agent and another anticancer agent through a simple assay with high sensitivity and better quantitativeness.

Disclosure of the Invention

Under the above circumstances, the present inventors have studied whether sensitivity to an anticancer agent can be determined through a single measurement by use of the widely employed existing DNA microarray method, and have identified the following problems.

Briefly, in use of the widely employed DNA microarray method, since the species of potential target genes counts several hundred to several dozens of thousands, it is considered to include those genes that are irrelevant to sensitivity to drugs, as conjectured from action mechanisms of currently available anticancer agents. As a result, analysis would require extra work, or genes which are closely related to the mechanisms of action of the anticancer agent may be overlooked or given less weight than they should be.

Moreover, in the case of the widely employed DNA microarray method, the quantity (concentration) of DNA to be spotted on a support carrier (e.g., nylon membrane, glass plate) is usually prefixed, and therefore, quantification may sometimes be unsuccessful because of extremely low expression level, despite the gene being closely related to sensitivity to the anticancer agent.

Thus, the present inventors have set out to improve the existing DNA microarray method. Firstly, they have narrowed the range of target genes to dozens to several hundreds of genes centering on those which are considered to be associated with mechanisms of action of an antimetabolite-type anticancer agents and other anticancer agents to be used in combination therewith. Next, through a homology-based search performed using databases, not only those genes determined through calculation but also those actually confirmed to have specificity through Northern hybridization, are determined to be target fragments. The thus-created DNA array in which fragments selected as described above are immobilized on the substrate has been found to be capable of providing judgments, by a single test with high sensitivity, with respect to an antimetabolite-type anticancer agent or a combination use of such an anticancer agent and another anticancer agent, thus leading to the present invention.

Accordingly, the present invention provides a DNA array for measuring sensitivity to an antimetabolite-type anticancer agent or to a combination of such an anticancer agent and another anticancer agent, characterized by comprising a substrate and target gene fragments attached to the substrate, as defined by claim 1. The target gene fragments are of at least 13 gene species selected by performing the following two steps 1) and 2):

1) selecting fragments having high target-gene specificity through a homology search using a database, and
2) performing Northern hybridization to RNA collected from tumor cells, using the fragments selected in step 1) as probes, to thereby confirm target-gene specificity.

The present invention also provides a method for measuring sensitivity of a body fluid specimen or tissue specimen of a cancer patient to an antimetabolite-type anticancer agent or to a combination of such an anticancer agent and another anticancer agent, characterized by comprising hybridizing the DNA array with labeled cDNA probes synthesized through use, as a template, of mRNA obtained from the specimen.

Brief Description of the Drawings

Fig. 1 shows universal primer sequences and the structure of a clone to which a target fragment is incorporated.
Fig. 2 contains actual images obtained from Northern hybridization, showing that in some cases different specificities result when DNA fragments serving as templates in the synthesis of probes are determined in different regions of a gene.
Fig. 3 shows the correlation between total RNA quantity and the measurements obtained in Northern hybridization.
Fig. 4 contains charts showing diagnosis quality provided by the array of the present invention, assessed in comparison with Northern hybridization.

Fig. 5 shows the correlation between TS-1 sensitivity and expression level, found in 11 types of xenografts, and contains graphs in connection with four genes which among 52 types of genes exhibited higher correlation.
Fig. 6 shows the correlation between TS-1 sensitivity and TS expression level, found in the 11 types of xenografts.

Best Mode for Carrying Out the Invention

The DNA array of the present invention is contemplated to be used in determining sensitivity to an antimetabolite serving as an anticancer agent (may be referred to as an antimetabolite-type anticancer agent), or in determining sensitivity to a combination of such an anticancer agent and another anticancer agent. Examples of the antimetabolite-type anticancer agent include 5-fluorouracil anticancer agents such as tegafur, 5-fluorouracil, tegafur·uracil, tegafur·gimeracil·oteracil potassium, carmofur, capecitabine, and furtulon; mercaptopurine anticancer agents such as 6-mercaptopurine and mercaptopurine riboside; anticancer cytosine analogues such as cytarabine, enocitabine, and gemcitabine; and methotrexate. Of these, 5-fluorouracil anticancer agents are particularly preferred. Examples of the mentioned "another anticancer agent" to be used in combination with the antimetabolites include platinum-complex-based anticancer agents such as cisplatin; topoisomerase inhibitors such as CPT-11 and VP-16; and taxane anticancer agents such as docetaxel and paclitaxel.
The measurement of sensitivity, as used in the present invention, refers to determination or judgment in terms of balance between efficacy of the anticancer agent on a patient and adverse side effects; decision of appropriate combination therapy, determination of appropriate administration scheme (dose, drug administration regimen), etc.
The target genes of the present invention are those which are considered to be associated with sensitivity, and consist of at least 13 species of gene, including at least two different species from each of the following groups: a group of genes coding for nucleic acid metabolism-related enzymes, a group of genes coding for gene-repair-associated enzymes, a group of genes coding for drug resistance-related factors, and a group of housekeeping genes.
Examples of the nucleic acid metabolism-related enzymes include thymidylate synthase (TS), dihydropyrimidine dehydrogenase (DPD), orotate phosphoribosyltransferase (OPRT) (uridine monophosphate synthetase (UMPS)), thymidine phosphorylase (TP), thymidine kinase 1 (TK1), ribonucleoside-diphosphate reductase M1 subunit (RRM1), ribonucleoside-diphosphate reductase M2 subunit (RRM2), uridine cytidine kinase 2 (UCK2), uridine phosphorylase (UP), cytidine deaminase (CDA), 5'nucleotidase (NT5), IMP dehydrogenase 1 (IMPD), methylenetetrahydrofolate dehydrogenase (MTHFD1), RNA polymerase 2 (RP2), uridine monophosphate kinase (UMPK), CTP synthase (CTPS), deoxycytidylate deaminase (DCD), deoxycytidine kinase (DCK), phosphoribosyl pyrophosphate synthetase (PRPS), hypoxanthine phosphoribosyltransferase 1 (HPRT1), folylpolyglutamate synthetase (FPGS), nucleoside diphosphate kinase A (NDKA), nucleoside diphosphate kinase B (NDKB), adenine phosphoribosyltransferase (APRT), and adenosine kinase (AK). Among the genes coding for these enzymes, preferably, at least genes coding for TS, DPD, ORRT, TP, and TK1 are employed.
Examples of the gene-repair-associated enzymes include DNA excision repair protein ERCC1 (ERCC1), uracil-DNA glycosylase (UDG), poly(ADP-ribose) polymerase (PARP), DNA ligase I (LIG1), DNA ligase III (LIG3), DNA ligase IV (LIG4), DNA polymerase $\beta$ (POLB), DNA polymerase $\delta$ (POLD), and DNA-repair protein XRCC1 (XRCC1). Among the genes coding for these enzymes, preferably, at least genes coding for ERCC1 and UDG are employed.
Examples of drug resistance-related factors include topoisomerase 1 (TOP1), P-glycoprotein (MDR1), equilibrative nucleoside transporter 1 (ENT1), multidrug resistance-associated protein 1 (MRP1), topoisomerase 2$\alpha$ (TOP2A), topoisomerase 2$\beta$ (TOPB), heatshockprotein 27 (Hsp27), and equilibrative nucleoside transporter 2 (ENT2). Among the genes coding for these factors, preferably, at least genes coding for TOP1, MDR1, ENT1, and MRP1 are employed.
Examples of the housekeeping genes include genes coding for glyceraldehyde-3-phosphate dehydrogenase (GAPDH), $\beta$-actin (ACTB), and 40S ribosomal protein S9 (RSP9). Preferably, at least two out of GAPDH gene, ACTB gene, and RSP9 gene are employed. These genes are used as internal standards.
Example of other genes include those coding for E2F1, p53, VEGF $\beta$, integrin $\alpha$3, Mn SOD, Cu/Zn SOD, or proliferating cell nuclear antigen (PCNA).
Desirably, fragments from all the mentioned target genes are immobilized. In view of ensuring achievement of more efficient measurement of sensitivity, fragments of at least 13 different genes in total are immobilized, including the following 11 genes; i.e., genes coding for TS, DPD, OPRT, TP, TK1, ERCC1, UDG, TOP1, MDR1, ENT1, and MRP1, as well as two or more of the genes coding for GAPDH, ACTB, or RSP9.
Regarding sequences of these target genes, a homology search is performed utilizing databases, whereby fragments which have high specificity to respective target genes are selected (Step 1).
The homology search may be carried out by use of, for example, Blast Search. Fragments may be designed as follows: GC content = 40 to 60%; reduced number of duplicate sequences; Tm = 75 to 85°C, size = 200 to 600 bp.
RNA obtained from tumor cells are subjected to Northern hybridization, using the fragments designed in step 1, whereby specificity is confirmed (step 2). Theoretically, the fragments designed in step 1 have high specificity to target genes. However, in reality, cross-hybridization occurs at high probability. In fact, only 10 to 20% of the fragments selected in step 1 are confirmed to have specificity in step 2.

In step 2, firstly, PCR amplification is performed through use of a cDNA library as a template, whereby a DNA fragment is obtained. Next, while the thus-obtained DNA fragment is used as a template, radioactive probes are enzymatically synthesized, and by use of a membrane bearing blots of total RNA samples prepared from various tumor cells, Northern hybridization is performed, whereby specificity is checked. Briefly, presence of specificity is confirmed when a signal that corresponds to the size of mRNA transcribed from a certain gene is detected (the size information can be obtained from literature or database searches) and almost no other signals are detected (which means almost no cross-hybridization).

If a DNA fragment is found to have insufficient specificity, by use of another region of a corresponding gene another DNA fragment is designed, and then the re-designed fragment is subjected to Northern hybridization. This procedure is repeated until sufficient specificity is obtained. Thus, regions of the DNA fragments to be arrayed can be optimized. Each of the thus-obtained target fragments is cloned into a plasmid (pCR2.1-TOPO).

In this connection, as primers to be used in PCR amplification of target fragments, universal primers are designed on the basis of the sequence of multiple cloning site of a cloning vector, so that all the target fragments can be amplified by use of a set of universal primers. Especially preferred universal primers are those having the nucleotide sequences of SEQ ID NOs: 1 and 2. Also, in order to minimize the volume of primers that migrate into the spotting solution, the universal primer volume is optimized. When these measures are taken, background of every spot from the target fragments can be suppressed, with reduced differences.

When the thus-obtained target fragments of respective target genes are immobilized onto a substrate (support), the DNA array of the present invention can be obtained. The substrate used herein is any suitable one known per se. For example, mention may be given of glass plate, plastic plate, membrane (nylon membrane, etc.), and beads. Preferably, a glass plate is used. Immobilization of the fragments onto the array may be achieved by use of conventional spotting means. For example, there may be employed the surface adhesion method (the Stanford method) as described in PCT Kohyo Publication No. 10-503841, in which a tip-split pin is dipped into a solution containing the target DNA, and tapped onto a support for transfer; the ink-jet piezoelectric discharge method, in which a solution containing the target DNA is jetted onto a support, under the same principle that used in an ink jet printer; and the photolithograph methodology, which makes use of photolithography techniques and the target DNA is directly synthesized on a support. Preferably, the Stanford method is used.

Preferably, mRNA of all the target genes is quantitated, and this quantitation can be achieved by modifying the amount of a target fragment to be immobilized in accordance with each gene's expression level obtained through Northern hybridization; i.e., the amount of target fragments to be immobilized on the substrate (support) is reduced for a gene of higher expression level, and increased for a gene of lower expression level.

In order to measure the sensitivity to the aforementioned anticancer agents by use of the thus-obtained DNA array of the present invention, the DNA array is hybridized with labeled cDNA probes synthesized by using, as a template, mRNA obtained from a body fluid specimen or tissue specimen collected form a cancer patient.

Examples of the body fluid specimen originating from a cancer patient include blood and urine. Examples of the tissue include cancerous tissue. Collection of mRNA from a specimen is carried out through a conventional method, and mRNA may be in the form of "as contained" in total RNA, or may be isolated from the total RNA. To prepare the labeled cDNA, mRNA is used as a template, and reverse transcription enzyme reaction is performed for labeling. Examples of labeling means include fluorescent substances and radioisotopes, with fluorescent substances being especially preferred.

Hybridization may be carried out under conventional conditions. Quantitation of hybridization may be achieved through quantitating the amount of labeled probes; for example, intensity of fluorescence.

In order to validate the quantitativeness of the DNA array of the present invention, the measurements in terms of expression level of 52 species of genes were compared with the measurements obtained from Northern hybridization. As a result, almost no discrepancy was found. Discernment of a gene that shows a unique expression characteristic (i.e., a gene exhibiting an expression level twice or more the average expression level) was able to be obtained at the same precision as attained by Northern hybridization.

## Examples

The present invention will next be described in more detail by way of examples, which should not be construed as limiting the invention thereto.

Example 1

(1) Preparation of target fragments

A. Selection and design of DNA fragments

As shown in Tables 2 and 3 below, 52 species of DNA fragments were selected as DNA fragments relating to 5-FU sensitivity, primarily from nucleic acid metabolism-related genes. In principle, the DNA fragments were designed such that each DNA fragment has a GC content of 40 to 60%, has almost no regions having the same nucleotide sequence as that of other DNA fragments of the same gene, has a Tm of 75 to 85°C, and has a size of 200 to 600 bp. In order to prevent cross-hybridization to the greatest possible extent, whether or not each of the DNA fragments had substantially no regions having the same nucleotide sequences as those of other DNA fragments in the same gene was investigated through a blast search by use of a database. By use of design-aiding software (Primer Express™, PE Biosystems), primers specific to each DNA fragment (specific primers [forward/reverse]), which were employed in PCR amplification, were designed so as to have a Tm of 59 to 61°C.

B. Synthesis and purification of DNA fragments

Each DNA fragment was amplified through PCR (30 cycles of treatment in total, each cycle consisting of thermal denaturation (94°C, 1 minute), annealing (60°C, 1 minute), and elongation (72°C, 1 minute)) while a human-derived cDNA library was used as a template, and by use of specific primers and an ExTaq™ (TaKaRa). The PCR product solution was subjected to purification through use of a spin column (Miniprep spin column, Aetna), and the purified product was eluted with distilled water.

(2) Northern hybridization

A. Preparation and purification of total RNA samples

A total RNA sample was extracted from each of the 14 types of human tumor cells listed in Table 1, through use of an RNeasy midi kit™ (QIAGEN) and eluted by distilled water. DNA fragments which might migrate in the solution were decomposed with DNase (37°C, 30 minutes), and proteins were removed by use of phenol/chloroform. The resultant solution was subjected to purification and condensation through ethanol precipitation, and the product was dissolved in distilled water, to thereby prepare a total RNA solution.

Table 1

| | Tumor type | Cell strain |
|---|---|---|
| 1 | Human gastric cancer | NUGC-3 |
| 2 | Human gastric cancer (FU-resistant) | NUGC-3/FU |
| 3 | Human large intestine cancer | DLD-1 |
| 4 | Human large intestine cancer (FU-resistant) | DLD-1/FU |
| 5 | Human large intestine cancer (FdUrd resistant) | DLD-1/FdUrd |
| 6 | Human large intestine cancer (F3dThd-resistant) | DLD-1/F3dThd |
| 7 | Human fibrosarcoma | HT1080 |
| 8 | Human fibrosarcoma (EUrd-resistant) | HT1080/EUrd |
| 9 | Human pancreatic carcinoma | MIAPaCa-2 |
| 10 | Human large intestine cancer | KM-12C |
| 11 | Human lung cancer | A549 |
| 12 | Human mammary cancer | MCF-7 |
| 13 | Human head and neck cancer | KB |
| 14 | Human ovarian cancer | TYK-nu |

B. Northern blotting

The total RNA sample (5 μg) which had been prepared from each of the above 14 types of human tumor cells was electrophoresed on 1-wt% denatured agarose gel. The gel was stained with ethidium bromide, and the resultant gel was photographed under irradiation of UV rays, to thereby confirm that the RNA molecules had not been decomposed. The RNA molecules in the gel were blotted on a nylon membrane through a capillary phenomenon, and the membrane was subjected to fixation (cross-linking) under irradiation with UV rays.

C. Preparation of probes

Probes labeled with [$\alpha$-$^{32}$P]dCTP were synthesized by use of each of the DNA fragments prepared in (1) above as a template, through random priming (rediprime™ II, Amersham Pharmacia). [$\alpha$-$^{32}$P]dCTP which had not been incorporated into the probes was removed through gel filtration (ProbeQuant™ G-50 micro spin column, Amersham Pharmacia).

D. Northern hybridization

Northern hybridization was performed by use of the blot and the probes prepared in (2)B and (2)C above, respectively. Specifically, pre-hybridization was performed by use of the blot in a hybridization buffer (Rapid-hyb Buffer, Amersham Pharmacia) for 30 minutes at 65°C, the probes which had been undergone thermal denaturation were added thereto, and hybridization was caused to proceed for two hours at 65°C. Subsequently, the resultant blot was washed (twice with a 2×SSC solution (0.15-mol/L NaCl / 0.15-mol/L trisodium citrate) containing 0.1-wt% SDS, once with a 1×SSC solution containing 0.1-wt% SDS, and twice with a 0.1×SSC solution containing 0.1-wt% SDS), and, in the dark, an imaging plate (Fujifilm) was exposed with the blot overnight. On the following day, the imaging plate was scanned by means of an imaging and analysis apparatus (STORM, Molecular Dynamics Inc.), and the obtained image data were stored. E. Evaluation and redesign of DNA fragments

Specificity of DNA fragments was evaluated from the images obtained through Northern hybridization. Specifically, when a signal was observed at a position corresponding to the size of mRNA of the gene of interest, and substantially no other signals were detected, the DNA fragment was determined to have specificity, whereas when no signal was observed at a position corresponding to the size of mRNA of the gene of interest, or when other signals were detected (cross-hybridization), the DNA fragment was determined to have poor specificity. In the latter case, another DNA fragment that would match the condition described in (1)A above was engineered in a different region of mRNA of the same gene. Subsequently, until such a redesigned DNA fragment was confirmed to have specificity, a cycle consisting of design, synthesis, and Northern hybridization was repeated (in some cases, the cycle was repeated six times), whereby the target fragment, the region of the gene to be fixed onto the DNA array of the present invention, was determined.

(3) Preparation of DNA array and hybridization

The following steps are based on the Stanford method, which was partially modified.

A. Cloning of target fragments

For preparing sufficient amounts of target fragments having good quality, use of cDNA library as a template is not preferred, since quality of cDNA library differs from lot to lot. Therefore, as shown in Fig. 1, each of the target fragments (the PCR products) was subjected to cloning through TA cloning by use of a plasmid (pCR-TOPO vector, Invitrogen), to thereby prepare a clone corresponding to the target fragment (52 species in total).

B. Preparation of target fragments

The target fragment was amplified in accordance with the method described in (1) above through use of the clone as a template. The employed primers were universal primers (SEQ ID NOS: 1 and 2). The PCR product was subjected to ethanol precipitation, and the collected precipitates were washed with ethanol and dissolved in distilled water. An aliquot of the solution was employed for calculation of concentration (through absorptiometry) and assay for determining purification (through electrophoresis on agarose gel). The remaining target fragment solution was dried at room temperature under reduced pressure, and then the fragment was dissolved in Micro Spotting Solution (BM) so as to have a concentration of 0.5 to 10 pmol/μL (Tables 2 and 3 show the concentration of each target fragment). Nucleotide sequences of the thus-obtained target fragments are shown by SEQ ID NOs: 3 to 54.

Table 2

| Name of gene | Abbreviation | GenBank Accession# | Amount of DNA on tip (pmol/μL) | Region [nt] (length [bp]) of target fragment | Number of Northern hybridization performed |
|---|---|---|---|---|---|
| **Nucleic acid metabolism-related enzyme** | | | | | |
| Thymidylate synthase | TS | NM_001071 | 1 | 1099-1466 (368) | 1 |
| Dihydropyrimidine dehydrogenase | DPD | U09178 | 10 | 1150-1537 (388) | 4 |
| Orotate phosphoribosyltransferase (uridine monophosphate synthetase) | OPRT (UMPS) | NM_000373 | 2 | 1299-1662 (364) | 1 |
| Thymidine phosphorylase | TP | M63193 | 10 | 490-797 (308) | 4 |
| Thymidine kinase 1 | TK1 | NM_003258 | 2.5 | 595-908 (314) | 2 |
| Ribonucleoside-diphosphate reductase M1 subunit | RRM1 | X59543 | 5 | 1669-2010 (342) | 3 |
| Ribonucleoside-diphosphate reductase M2 subunit | RRM2 | NM_001034 | 1 | 1840-2097 (258) | 3 |
| Uridine cytidine kinase 2 | UCK2 | AF236637 | 2.5 | 61-609 (549) | 3 |
| Uridine phosphorylase | UP | NM_003364 | 10 | 931-1314 (384) | 3 |
| Cytidine deaminase | CDA | L27943 | 5 | 304-605 (302) | 3 |
| 5' nucleotidase | NT5 | NM_002526 | 5 | 2800-3073 (274) | 3 |
| IMP dehydrogenase 1 | IMPD | NM_000883 | 5 | 1967-2220 (254) | 4 |
| Methylenetetrahydrofolate dehydrogenase | MTHFD1 | NM_005956 | 2.5 | 1768-2071 (304) | 3 |
| RNA polymerase 2 | RP2 | NM_000937 | 2.5 | 1457-1787 (331) | 6 |
| Uridine monophosphate kinase | UMPK | NM_016308 | 5 | 719-1003 (285) | 4 |
| CTP synthase | CTPS | NM_001905 | 2.5 | 2130-2394 (265) | 3 |
| Deoxycytidylate deaminase | DCD | NM_001921 | 5 | 1176-1543 (368) | 3 |
| Deoxycytidine kinase | DCK | NM_000788 | 5 | 542-963 (422) | 2 |
| Phosphoribosyl pyrophosphate synthetase | PRPS | D00860 | 5 | 1152-1473 (322) | 3 |
| Hypoxanthine phosphoribosyltransferase 1 | HPRT1 | NM_000194 | 2.5 | 824-1214 (391) | 2 |

(continued)

| Name of gene | Abbreviation | GenBank Accession# | Amount of DNA on tip (pmol/μL) | Region [nt] (length [bp]) of target fragment | Number of Northern hybridization performed |
|---|---|---|---|---|---|
| Folylpolyglutamate synthetase | FPGS | NM_004957 | 10 | 92-311 (220) | 3 |
| Nucleoside diphosphate kinase A | NDKA | X17620 | 0.5 | 299-662 (364) | 1 |
| Nucleoside diphosphate kinase B | NDKB | L16785 | 0.5 | 210-581 (372) | 1 |
| Adenine phosphoribosyltransferase | APRT | NM_000485 | 5 | 154-559 (406) | 1 |
| Adenosine kinase | AK | U33936 | 5 | 651-1019 (369) | 1 |

Table 3

| Name of gene | Abbreviation | GenBank Accession# | Amount of DNA on tip (pmol/μL) | Region [nt] (length [bp]) of target fragment | Number of Northern hybridization performed |
|---|---|---|---|---|---|
| **Gene repair-associated enzyme** | | | | | |
| DNA excision repair protein ERCC1 | ERCC1 | NM_001983 | 5 | 210-526 (317) | 2 |
| Uracil-DNA glycosylase | UDG | X15653 | 2.5 | 1553-1943 (391) | 1 |
| Poly(ADP-ribose) polymerase | PARP | NM_001618 | 2.5 | 2684-3093 (410) | 3 |
| DNA ligase I | LIG1 | NM_000234 | 5 | 1363-1671 (309) | 3 |
| DNA ligase III | LIG3 | X84740 | 10 | 1680-2034 (355) | 2 |
| DNA ligase IV | LIG4 | NM_002312 | 10 | 2088-2498 (411) | 4 |
| DNA polymerase β | POLB | NM_002690 | 5 | 110-444 (335) | 2 |
| DNA polymerase δ | POLD | NM_002691 | 5 | 1198-1459 (262) | 3 |
| DNA-repair protein XRCC1 | XRCC1 | NM_006297 | 10 | 898-1265 (368) | 3 |
| **Drug resistance-related factor** | | | | | |
| Topoisomerase 1 | TOP1 | J03250 | 2.5 | 1133-1456 (324) | 3 |
| P-glycoprotein | MDR1 | NM_000927 | 5 | 1617-1881 (265) | 3 |

(continued)

| Name of gene | Abbreviation | GenBank Accession# | Amount of DNA on tip (pmol/μL) | Region [nt] (length [bp]) of target fragment | Number of Northern hybridization performed |
|---|---|---|---|---|---|
| Equilibrative nucleoside transporter 1 | ENT1 | NM_004955 | 2.5 | 148-403 (256) | 3 |
| Multidrug resistance-associated protein 1 | MRP1 | L05628 | 10 | 3841-4239 (399) | 5 |
| Topoisomerase 2 α | TOP2A | NM_001067 | 2.5 | 3037-3389 (353) | 6 |
| Topoisomerase 2 β | TOP2B | X68060 | 2.5 | 3142-3515 (374) | 3 |
| Heat shock protein 27 | Hsp27 | NM_001540 | 1 | 323-534 (212) | 3 |
| Equilibrative nucleoside transporter 2 | ENT2 | AF034102 | 5 | 1941-2263 (323) | 3 |
| **Others** | | | | | |
| E2F1 | E2F1 | M96577 | 5 | 1014-1309 (296) | 5 |
| p53 | p53 | NM_000546 | 2.5 | 796-1130 (335) | 2 |
| VEGF β | VEGFB | U48801 | 10 | 97-375 (279) | 1 |
| Integrin α 3 | ITGA3 | NM_002204 | 2.5 | 2563-2930 (368) | 3 |
| Mn SOD | SOD2 | NM_000636 | 2.5 | 60-329 (270) | 1 |
| Cu/Zn SOD | SOD1 | X02317 | 2.5 | 53-312 (260) | 2 |
| Proliferating cell nuclear antigen | PCNA | NM_002592 | 1 | 108-423 (316) | 1 |
| **Housekeeping gene (internal standard)** | | | | | |
| Glyceraldehyde-3-phosphate dehydrogenase | GAPDH | X01677 | 0.5 | 453-785 (333) | 2 |
| β-actin | ACTB | NM_001101 | 0.5 | 820-1083 (264) | 2 |
| 405 ribosomal protein S9 | RSP9 | U14971 | 0.5 | 325-685 (361) | 1 |

C. Spotting and post-treatment

The target fragment which had undergone thermal denaturation for three minutes at 95°C was spotted onto a glass slide coated with poly-L-lysine, by means of a spotter (OmniGrid, GENEMACHINES). Subsequently, the target fragment was cross-linked with the glass slide under irradiation with UV rays, and the glass slide was placed in a rack and shaken in a blocking solution (8-wt% Block A in PBS) for 30 minutes, and the resultant slide was washed with a TE buffer and dried. The slide glass was stored in a desiccator in the dark until the time of use. Following the process, the DNA array of the present invention was obtained.

D. Preparation of fluorescence-labeled DNA probes

Reverse transcription reaction was performed through use of a total RNA sample prepared from tumor cells (through the same method as described in (2) above) as a template and primers specific to mRNA of each gene, to thereby prepare fluorescence-labeled probes. Reagents employed for labeling reaction are as follows.

- reverse transcriptase SuperscriptII (Gibco BRL)
- (reaction buffer, DTT)
- a primer mixture (a mixture of reverse primers specific to each of the 52 genes; the reverse primers are one of the specific primers employed in Referential Example 1)
- dATP, dGTP, dCTP, dTTP (Amersham Pharmacia)
- Cy3-dUTP (Amersham Pharmacia)
- Cy5-dUTP (Amersham Pharmacia)
- 0.5M EDTA
- 1N NaOH
- 1M Tris-HCl (pH 7.5)
- TE buffer

Specifically, a total RNA sample (30 μg) was mixed with a primer mixture (50 pmol each) and distilled water, and the volume of the resultant mixture was adjusted to 9 μL. The mixture was denatured for two minutes at 65°C and rapidly cooled on ice. A reaction buffer (1×), DTT (10 mM), dTTP (0.2 mM), dATP (0.5 mM), dGTP (0.5 mM), dCTP (0.5 mM), Cy3-dUTP or Cy5-dUTP (0.1 mM), and Superscript II (10 U/μL) were added thereto, and the total volume of the mixture was adjusted with distilled water to 20 μL (the concentrations in the parentheses refer to final concentrations). The mixture was allowed to react for 60 minutes at 42°C, and distilled water (20 μL), 0.5M EDTA (5 μL), and 1N NaOH (5 μL) were added thereto. The resultant mixture was incubated for 60 minutes at 65°C, whereby total RNA molecules were decomposed. The reaction mixture was neutralized with 1M Tris-HCl (25 μL). A TE buffer (200 to 400 μL) was added thereto, and the resultant mixture was desalted and concentrated through ultrafiltration (Microcon-30, Millipore) (in this step, reverse primers and Cy3-dUTP or Cy5-dUTP which had not been incorporated into probes were also removed). Ultimately, about 10 μL of a probe solution was obtained.

E. Hybridization

20×SSC (3 μL) and distilled water were added to the above fluorescence-labeled DNA probe solution, and the volume of the mixture was adjusted to 20 μL (final concentration: 3×SSC). The mixture was subjected to thermal denaturation for three minutes at 95°C, and the reaction mixture was left to stand at room temperature for cooling. 10-wt% SDS (2 μL) and distilled water were added to the resultant mixture, and the volume of the mixture was adjusted to 40 μL (final concentration: 0.5 wt%). The mixture was added dropwise to the DNA array prepared in the steps A to C, and a glass cover was gently placed onto the array (so as not to allow air inside). The DNA array was set in a hybridization chamber and incubated for 10 to 20 hours at 65°C. Thereafter, the DNA array was placed in a 2×SSC solution containing 0.2-wt% SDS, and the glass cover was gently removed. The DNA array was washed *in situ* for five minutes. Moreover, the DNA array was washed once with a 2×SSC solution containing 0.2-wt% SDS and twice with a 0.2×SSC solution containing 0.2-wt% SDS, and rinsed twice with 0.2×SSC (all of these washing steps were performed at room temperature). The DNA array was placed in a rack and centrifuged for 20 seconds at 600 rpm, to thereby remove water. The DNA array was dried at room temperature, and fluorescent signals from the DNA array were measured by means of a DNA microarray fluorescence scanner (GenePix, Axon).

Test 1: Specificity of DNA fragments

Specificity of DNA fragments employed as templates for synthesis of probes in Northern hybridization differed from fragment to fragment, although the fragments were contained in the same gene. Fig. 2 shows some of the test results, using XRCC1 and E2F1. In XRCC1, a region of 898 to 1265 nt exhibited good results (strong signals were detected at a position corresponding to the size of mRNA of the gene of interest), and a region of 187 to 494 nt was determined not to be suitable, since a large number of signals indicating cross-hybridization were observed. Similarly, in E2F1, a region of 1014 to 1309 nt exhibited good results, and a region of 788 to 1087 nt was determined not to be suitable. When regions of other genes were investigated, various levels of specificity were also observed, although the levels differed from gene to gene. A DNA fragment which exhibited the highest specificity in DNA fragments of gene of interest was selected in the final step and was employed as a target fragment.

Test 2: Performance of Northern hybridization in terms of quantitativeness

Performance of DNA arrays in terms of quantitativeness is confirmed by comparison with Northern hybridization. Accordingly, in order to confirm the performance of the measurement system of Northern hybridization employed by the present inventors (Example 1(2)), correlation of the RNA level and measurements (signal intensity) was investigated.

A total RNA sample derived from each of the above 14 types of human tumor cells was electrophoresed on a denatured agarose gel and blotted on a nylon membrane. Northern hybridization was performed through use of probes synthesized from target DNA of GAPDH (glyceraldehydes-3-phosphate dehydrogenase) (through the method described in Example 1(2)). Analysis was performed by means of imaging and analysis software (ImageQuant, Molecular Dynamics Inc), and intensity of GAPDH mRNA signals was measured.

Fig. 3 shows the relationship between the amount of the electrophoresed total RNA sample and measurements of Northern hybridization. The results confirmed that a linear relationship exists between the amount of the total RNA sample and signal intensity ($r=0.95$, $p<0.01$), indicating that the measurement system employed provides high performance in terms of quantitativeness.

Test 3: Effect of universal primers on background

As shown in Fig. 1, universal primers (forward (pCR-F)/reverse (pCR-R)) were designed on the basis of the sequence of a multicloning site of vector. Since all 52 target fragments can be amplified at a constant rate by using universal primers, target fragments can be readily provided in sufficient amounts as compared with the case of specific primers.

In order to determine the effect of primers which may migrate into target fragments upon spotting on measurement background, universal primers (5 spots) and specific primers (primers corresponding to each of about 20-bp sequences at both ends of 5 target DNA fragments [TS, DPD, OPRT, LIG4, and GAPDH] listed in Tables 2 and 3, 1 spot each) were spotted in equal amounts onto glass slides. The glass slides were subjected to the hybridization procedure described in Example 1(3), and signal intensity was measured.

The mean value, standard deviation (SD), and coefficient of variation (CV) of signal intensities obtained from the universal primers (5 spots) were found to be 27.3, 6.5, and 24.0%, respectively. The mean value, standard deviation (SD), and coefficient of variation (CV) of signal intensities obtained from the specific primers (5 spots in total) were found to be 22.9, 12.0, and 52.4%, respectively. Signal intensities from both primers were found to be of substantially the same level, and signal intensities obtained from the universal primers had lower SD and CV, indicating that the universal primers exert less effect on difference in background level than the specific primers.

In addition, in order to reduce background to the lowest level as possible, the minimum amount of the universal primers required to amplify a target fragment was studied. PCR was performed through use of forward and reverse primers. The amount of each primer was 10, 20, 30, 40, and 50 pmol. The PCR products were electrophoresed on agarose gel. When primers were used in amounts of 20 pmol or less, the amount of PCR product was found to be reduced. Therefore, the minimum amount of the universal primers required to amplify a target fragment was determined to be 30 pmol.

Example 2 Measurement of expression level of various genes in human tumor cells

(1) Measurement through Northern hybridization

In the step of determining target fragments to be placed onto the DNA array, the expression level of each of the 52 genes was determined through analysis of blot images obtained through Northern hybridization of the above 14 different human tumor cells (see Example 1(2)). The expression level is represented by the value relative to the mean value of the expression levels of three housekeeping genes (GAPDH, ACTB, and RSP9).

(2) Measurement by use of DNA array

A DNA array onto which the 52 DNA target fragments were immobilized was prepared, and hybridization was performed through use of fluorescent DNA probes prepared from the total RNA samples which had been extracted from the 14 types of cells and employed in Northern hybridization. The expression level of each gene was measured (through the same method as described in Example 1 (3)). The expression level is represented by the value relative to the mean value of the expression levels of three housekeeping genes (GAPDH, ACTB, and RSP9).

(3) Correlation of the results obtained through Northern hybridization with the results obtained through DNA array

In order to determine whether the expression levels of each of the 52 species of genes determined through Northern hybridization in the above 14 species of human tumor cells were correlated with those determined by use of DNA arrays, regression analysis was performed (Table 4). The degree of correlation (correlation coefficient) differed from gene species to gene species, and significant correlation was confirmed in 20 species of genes at a significance level of less than 5%. Some degree of correlation was confirmed in nine species of genes, although the degree was not significant (p<0.1). Twelve species of genes containing three species of housekeeping genes (MTHFD1, SOD1, AK, E2F1, POLD, LIG3, RSP9, HPRT1, ACTB, UDG, GAPDH, and LIG1) were found to have only a low degree of correlation or were found impossible to evaluate, since difference in expression level between cells were small; in other words, the normal distribution was not obtained.

Table 4

| Abbreviation | R (correlation coefficient) | P-value | Correlation | Significance (P<0.05) |
|---|---|---|---|---|
| ERCC1 | 0.90 | 9.4E-06 | High | Yes |
| p53 | 0.90 | 1.1E-05 | High | Yes |
| MDR1 | 0.90 | 1.3E-05 | High | Yes |
| DPD | 0.89 | 2.2E-05 | High | Yes |
| CDA | 0.87 | 5.0E-05 | High | Yes |
| TS | 0.85 | 1.1E-04 | High | Yes |
| PRP5 | 0.85 | 1.2E-04 | High | Yes |
| NDKB | 0.80 | 6.5E-04 | Relatively high | Yes |
| POLB | 0.79 | 7.3E-04 | Relatively high | Yes |
| XRCC1 | 0.77 | 1.3E-03 | Relatively high | Yes |
| MRP1 | 0.76 | 1.4E-03 | Relatively high | Yes |
| ENT2 | 0.75 | 2.0E-03 | Relatively high | Yes |
| APRT | 0.75 | 2.2E-03 | Relatively high | Yes |
| Hsp27 | 0.67 | 8.2E-03 | Relatively high | Yes |
| VEGFB | 0.60 | 2.2E-02 | Relatively high | Yes |
| TOP2B | 0.57 | 3.3E-02 | Medium | Yes |
| LIG4 | 0.57 | 3.4E-02 | Medium | Yes |
| ENT1 | 0.55 | 4.3E-02 | Medium | Yes |
| RRM2 | 0.54 | 4.6E-02 | Medium | Yes |
| IMPD | 0.54 | 4.8E-02 | Medium | Yes |
| NDKA | 0.53 | 5.3E-02 | Medium | No |
| RP2 | 0.51 | 6.2E-02 | Medium | No |
| TP | 0.50 | 7.1E-02 | Medium | No |
| RRM1 | 0.49 | 7.7E-02 | Medium | No |
| UCK2 | 0.45 | 1.1E-01 | Medium | No |
| FPGS | 0.44 | 1.2E-01 | Medium | No |
| CTPS | 0.44 | 1.2E-01 | Medium | No |
| UMPS(OPRT) | 0.42 | 1.3E-01 | Medium | No |
| SOD2 | 0.42 | 1.4E-01 | Medium | No |

(continued)

| Abbreviation | R (correlation coefficient) | P-value | Correlation | Significance (P<0.05) |
|---|---|---|---|---|
| UP | 0.39 | 1.6E-01 | Relatively low | No |
| NT5 | 0.34 | 2.3E-01 | Relatively low | No |
| TK1 | 0.34 | 2.4E-01 | Relatively low | No |
| DCK | 0.33 | 2.5E-01 | Relatively low | No |
| TOP2A | 0.32 | 2.7E-01 | Relatively low | No |
| DCD | 0.27 | 3.5E-01 | Relatively low | No |
| ITGA3 | 0.26 | 3.7E-01 | Relatively low | No |
| PARP | 0.24 | 4.1E-01 | Relatively low | No |
| TOP1 | 0.23 | 4.3E-01 | Relatively low | No |
| PCNA | 0.23 | 4.3E-01 | Relatively low | No |
| UMPK | 0.21 | 4.6E-01 | Relatively low | No |
| LIG1 | 0.06 | 8.3E-01 | Low | No |
| MTHFD1 | 0.40 | 1.6E-01 | Impossible to evaluate | No |
| SOD1 | 0.39 | 1.6E-01 | Impossible to evaluate | No |
| AK | 0.37 | 1.9E-01 | Impossible to evaluate | No |
| E2F1 | 0.29 | 3.2E-01 | Impossible to evaluate | No |
| POLD | 0.28 | 3.3E-01 | Impossible to evaluate | No |
| LIG3 | 0.27 | 3.4E-01 | Impossible to evaluate | No |
| RSP9 | 0.26 | 3.7E-01 | Impossible to evaluate | No |
| HPRT1 | 0.25 | 3.8E-01 | Impossible to evaluate | No |
| ACTB | 0.20 | 5.0E-01 | Impossible to evaluate | No |
| UDG | 0.17 | 5.7E-01 | Impossible to evaluate | No |
| GAPDH | 0.14 | 6.4E-01 | Impossible to evaluate | No |

(4) Performance of DNA array in terms of judgment

When such a tool used for the purpose of diagnosis (proper use of a drug) is employed in actual clinical sites, whether a sample is positive or negative is generally determined on the basis of threshold. For example, in research in which TS or DPD mRNA level determined through RT-PCR is used as an index which indicates proper use of 5-FU, a threshold has been set for determining that a level is high or low (Clinical Cancer Research, 6, 1322-1327, 2000). The performance of the DNA array of the present invention was determined through comparison with Northern hybridization in the following manner.

The above 14 species of human tumor cells were treated as clinical specimens. The expression level of each of the 52 species of genes in each type of cells was measured through use of the DNA array of the present invention or through Northern hybridization (in each method, 52 (genes) $\times$ 14 (cells) = 728 points were measured and calculated). From measurements obtained through use of the DNA array of the present invention, the median value of the expression levels of each gene in the 14 species of cells was calculated. The relative expression level (the ratio of expression level of each of the 14 species of cells to the median value (base)) was calculated. The threshold was set as 2-fold. When the relative expression level went beyond the threshold value ($\geq$2 or $\leq$0.5), the cell was determined to be "positive," whereas when the relative expression level did not exceed the threshold value, the cell was determined to be "negative." On the assumption that results determined through Northern hybridization were highly reliable, when results determined by use of the DNA array of the present invention were identical with those determined through Northern hybridization, the results were evaluated as "true," whereas when results determined by use of the DNA array of the present invention were different from those determined through Northern hybridization, the results were evaluated as "faulty." As shown in Fig. 4, in the positive and negative results, the percentage of data evaluated "true" was found to be as high as 82.3%. In 75 points which had been determined as positive through use of the DNA array of the present invention, 77.3% (58/75) of the points presented the same results as those of Northern hybridization. In addition, there were no cases in which the measurements and results of the DNA array were inconsistent with those of Northern hybridization (for example, there were no cases in which both results were true positive but the relative expression level through Northern hybridization (e.g., 0.3) was quite different from those obtained through use of the DNA array of the present invention (e.g., 2.5)). Thus, the DNA array of the present invention was found to present substantially the same level of performance as that of Northern hybridization.

(5) Detecting ability of DNA array

In Test 4 (4), among the tested 728 points (52 (genes) × 14 (cells)), 62 points were found to be impossible to measure. None of the 62 points could not be detected through Northern hybridization. Therefore, the results indicate that, as compared with Northern hybridization, the DNA array of the present invention presents the same level or a higher level of detecting ability.

Example 3 <u>Correlation between TS-1 sensitivity and expression level of various genes in transplantable human tumor cells</u>

(1) TS-1 sensitivity test

The DNA array of the present invention may be clinically applied to, for example, determining the genes that are important as sensitivity regulating factors, by analyzing correlation between expression levels of the 52 genes in cancer tissue and antitumor effect of a 5-FU anticancer agent.

In this test, as a test model for determining anticancer effect, TS-1 sensitivity of 11 species of transplantable human tumor cells (xenografts, derived from gastric cancer (4 types), large intestine cancer (3 types), lung cancer (2 types), and mammary cancer (2 types)) was tested (TS-1, a 5-FU anticancer agent which has been developed by Taiho Pharmaceutical Co., Ltd., is a composition-containing agent containing tegafur (5-FU prodrug), gimeracil (a DPD inhibitor), and oteracil potassium (an orotate phosphoribosyl transferase inhibitor) at a ratio by mole 1:0.4:1). Each xenograft was subcutaneously transplanted into nude mice at the back thereof. When the transplanted site was expanded to 100 to 200 $mm^3$, the mice were divided into two groups; i.e., a control group (a group to which no drug was administered) and a TS-1 administered group (6 animals/group). From the following day, TS-1 was perorally administered to mice of the TS-1 administered group once a day for 14 successive days at a dose of 10 mg/kg/day (FT). On the day following the final administration, the volumes of the tumors were measured, and the volumes of the TS-1 administered group were compared with those of the control group. Tumor multiplication inhibition rate (IR) was determined. IR was calculated by the following equation.

IR(%)=(1-[Relative tumor volume of the TS-1 administered group]/[Relative tumor volume of the control group])×100

(Relative tumor volume)=(tumor volume upon judgement)/(tumor volume upon grouping)

(2) Measurement by use of the DNA array of the present invention

The expression levels of the 52 species of genes were determined through use of total RNA samples prepared from the 11 species of xenografts. The test was performed in accordance with the method described in Example 2. The expression level is represented by the value relative to the mean value of the expression levels of three species of housekeeping genes (GAPDH, ACTB, and RSP9).

(3) Correlation between the expression level in 11 species of xenografts and TS-1 sensitivity

In order to determine whether the expression level of the 52 species of the genes in the 11 species of xenograft, determined in the studies (1) and (2) above, is correlated with TS-1 sensitivity (IR, %), regression analysis was performed (52 (genes) × 11 (cells)). As a result, four species of the gene (UDG, PCNA, TS, and TK1) were found to have an absolute value of correlation coefficient (P-value: Pearson's product-moment correlation coefficient) higher than 0.5 (Fig. 5). P-value was found to be 0.05 or higher and lower than 0.1. Thus, the data were not statistically significant. However, since N is as small as 11, from the regression line, the expression level is confirmed to be substantially correlated with the sensitivity. It should be noted that these four species of genes contain TS. TS is a representative sensitivity regulating factor for 5-FU anticancer agent, and both basic research and clinical research have revealed that the higher the TS expression level, the lower the effect exhibited by a 5-FU anticancer agent. In the study of the present invention, TS expression level was found to have a negative correlation with antitumor effect of TS-1 (r=-0.53). The results coincide with the previous finding. In addition, as a confirmation test, the TS expression level in the 11 species of cells (relative expression level against GAPDH) was measured through real time RT-PCR, which is considered to provide considerably high measurement accuracy, to thereby analyze correlation between TS-1 sensitivity and the TS expression level. The correlation coefficient was found to be -0.65; i.e., a significant correlation (P=0.030) was confirmed (Fig. 6). These data indicate that the DNA array of the present invention is useful for determining candidates of sensitivity regulating factors of a 5-FU anticancer agent from the 52 species of gene. When N increases, clinical specimens may be used to determine sensitivity regulating factors of a 5-FU anticancer agent, and when the expression level of a selected gene was determined through analysis, the expression level may be used as an index which indicates proper use of 5-FU anticancer agent.

Industrial Applicability

The present invention achieves convenient and highly quantitative measurement of expression levels of several tens to several hundreds of gene species contained in a specimen in a single measurement. When expression patterns of genes related to action mechanisms of an antimetabolite-type anticancer agent or a combination of such an agent and another anticancer agent are analyzed through use of the assay method of the present invention in a test specimen (for example, total RNA extracted from peripheral monocytes or tumor tissue of a cancer patient), results obtained from such an analysis can be employed as indices for proper use of the anticancer agent.

## Claims

1. A DNA array for measuring sensitivity to an antimetabolite-type anticancer agent or to a combination of such an anticancer agent and another anticancer agent, **characterized by** comprising a substrate and at least the following gene fragments (A), (B), (C) and (D) attached to the substrate:

   (A) a thymidylate synthase gene fragment consisting of a nucleotide sequence of SEQ ID NO: 3, a dihydropyrimidine dehydrogenase gene fragment consisting of a nucleotide sequence of SEQ ID NO: 4, an orotate phosphoribosyltransferase gene fragment consisting of a nucleotide sequence of SEQ ID NO: 5, a thymidine phosphorylase gene fragment consisting of a nucleotide sequence of SEQ ID NO: 6, and a thymidine kinase 1 gene fragment consisting of a nucleotide sequence of SEQ ID NO: 7;
   (B) a DNA excision repair protein ERCC1 gene fragment consisting of a nucleotide sequence of SEQ ID NO: 28 and a uracil-DNA glycosylase gene fragment consisting of a nucleotide sequence of SEQ ID NO: 29;
   (C) a topoisomerase 1 gene fragment consisting of a nucleotide sequence of SEQ ID NO: 37, a P-glycoprotein gene fragment consisting of a nucleotide sequence of SEQ ID NO: 38, an equilibrative nucleoside transporter 1 gene fragment consisting of a nucleotide sequence of SEQ ID NO: 39, and a multidrug resistance-associated protein 1 gene fragment consisting of a nucleotide sequence of SEQ ID NO: 40; and
   (D) two or more gene fragments selected from a housekeeping gene group consisting of a glyceraldehyde-3-phosphate dehydrogenase gene fragment consisting of a nucleotide sequence of SEQ ID NO: 52, a beta-actin gene fragment consisting of a nucleotide sequence of SEQ ID NO: 53, and a 40S ribosomal protein S9 gene fragment consisting of a nucleotide sequence of SEQ ID NO: 54.

2. The DNA array according to claim 1, wherein each of the gene fragments has a size of 200 to 600 bp.

3. The DNA array according to claim 1, wherein said DNA array further comprises a proliferating cell nuclear antigen gene fragment consisting of a nucleotide sequence of SEQ ID NO: 51 attached to said substrate.

4. A method for measuring sensitivity of a body fluid specimen or tissue specimen of a cancer patient to an antimetabolite-type anticancer agent or to a combination of such an anticancer agent and another anticancer agent, **characterized by** comprising hybridizing a DNA array as recited in claim 1, 2 or 3 with labeled cDNA probes synthesized through use, as a template, of mRNA obtained from the specimen.

5. The method according to claim 4, wherein the amount of a respective fragment attached to the substrate is reduced for a gene of higher expression level and increased for a gene of lower expression level of a target genes in tumor cells.

6. The method according to claim 4, wherein said antimetabolite-type anticancer agent is TS-1.

7. A method of producing a DNA array for measuring sensitivity to an antimetabolite-type anticancer agent or to a combination of such an anticancer agent and another anticancer agent, **characterized by** comprising a substrate and at least the following gene fragments (A), (B), (C) and (D) attached to the substrate:

(A) a thymidylate synthase gene fragment consisting of a nucleotide sequence of SEQ ID NO: 3, a dihydropyrimidine dehydrogenase gene fragment consisting of a nucleotide sequence of SEQ ID NO: 4, an orotate phosphoribosyltransferase gene fragment consisting of a nucleotide sequence of SEQ ID NO: 5, a thymidine phosphorylase gene fragment consisting of a nucleotide sequence of SEQ ID NO: 6, and a thymidine kinase 1 gene fragment consisting of a nucleotide sequence of SEQ ID NO: 7;
(B) a DNA excision repair protein ERCC1 gene fragment consisting of a nucleotide sequence of SEQ ID NO: 28 and a uracil-DNA glycosylase gene fragment consisting of a nucleotide sequence of SEQ ID NO: 29;
(C) a topoisomerase 1 gene fragment consisting of a nucleotide sequence of SEQ ID NO: 37, a P-glycoprotein gene fragment consisting of a nucleotide sequence of SEQ ID NO: 38, an equilibrative nucleoside transporter 1 gene fragment consisting of a nucleotide sequence of SEQ ID NO: 39, and a multidrug resistance-associated protein 1 gene fragment consisting of a nucleotide sequence of SEQ ID NO: 40; and
(D) two or more gene fragments selected from a housekeeping gene group consisting of a glyceraldehyde-3-phosphate dehydrogenase gene fragment consisting of a nucleotide sequence of SEQ ID NO: 52, a beta-actin gene fragment consisting of a nucleotide sequence of SEQ ID NO: 53, and a 40S ribosomal protein S9 gene fragment consisting of a nucleotide sequence of SEQ ID NO: 54.

**8.** The method according to claim 7, wherein said DNA array further comprises a proliferating cell nuclear antigen gene fragment consisting of a nucleotide sequence of SEQ ID NO: 51.

**9.** The method according to claim 7, wherein the amount of a respective fragment attached to the substrate is reduced for a gene of higher expression level and increased for a gene of lower expression level of a target genes in tumor cells.

**Patentansprüche**

**1.** DNA-Array zur Messung der Sensitivität auf ein Antikrebsmittel vom Antimetabolit-Typ oder auf eine Kombination eines solchen Antikrebsmittels und eines anderen Antikrebsmittels, **dadurch gekennzeichnet, dass** es ein Substrat und mindestens die folgenden Genfragmente (A), (B), (C) und (D), die an das Substrat gebunden sind, umfasst:

(A) ein Thymidylatsynthase Genfragment bestehend aus einer Nukleotidsequenz von SEQ ID NO: 3, ein Dihydropyrimidindehydrogenase Genfragment bestehend aus einer Nukleotidsequenz von SEQ ID NO: 4, ein Orotatphosphoribosyltransferase Genfragment bestehend aus einer Nukleotidsequenz von SEQ ID NO: 5, ein Thymidinphosphorylase Genfragment bestehend aus einer Nukleotidsequenz von SEQ ID NO: 6 und ein Thymidinkinase-1 Genfragment bestehend aus einer Nukleotidsequenz von SEQ ID NO: 7;
(B) ein DNA Excision Repair Protein ERCC1 Genfragment bestehend aus einer Nukleotidsequenz von SEQ ID NO: 28 und ein Uracil-DNA-Glycosylase Genfragment bestehend aus einer Nukleotidsequenz von SEQ ID NO: 29;
(C) ein Topoisomerase 1 Genfragment bestehend aus einer Nukleotidsequenz von SEQ ID NO: 37, ein P-Glycoprotein Genfragment bestehend aus einer Nukleotidsequenz von SEQ ID NO: 38, ein equilibrative nucleoside Transporter 1 Genfragment bestehend aus einer Nukleotidsequenz von SEQ ID NO: 39, und ein multidrug resistence associated Protein-1 Genfragment bestehend aus einer Nukleotidsequenz von SEQ ID NO: 40; und
(D) zwei oder mehr Genfragmente ausgewählt aus einer Haushaltsgengruppe bestehend aus einem Glycerinaldehyd-3-phosphatdehydrogenase Genfragment bestehend aus einer Nukleotidsequenz von SEQ ID NO: 52, einem Beta-Actin Genfragment bestehend aus einer Nukleotidsequenz von SEQ ID NO: 53 und einem 40S ribosomalen Protein S9 Genfragment bestehend aus einer Nukleotidsequenz von SEQ ID NO: 54.

**2.** Das DNA-Array gemäß Anspruch 1, worin jedes der Genfragmente eine Größe von 200 bis 600 bp aufweist.

**3.** Das DNA-Array gemäß Anspruch 1, worin das DNA-Array ferner ein proliferating cell nuclear antigen Genfragment bestehend aus einer Nukleotidsequenz von SEQ ID NO: 51 aufweist, das an das Substrat gebunden ist.

**4.** Verfahren zur Messung der Sensitivität einer Probe einer Körperflüssigkeit oder einer Gewebeprobe eines Krebspatienten auf ein Antikrebsmittel vom Antimetabolit-Typ oder auf eine Kombination eines solchen Antikrebsmittels und eines anderen Antikrebsmittels, **dadurch gekennzeichnet, dass** es ein Hybridisieren eines DNA-Array gemäß einem der Ansprüche 1, 2 oder 3 mit markierten cDNA Sonden umfassst, die unter Verwendung von aus der Probe erhaltener mRNA als Templat synthetisiert wurden.

5. Das Verfahren gemäß Anspruch 4, worin die Menge eines betreffenden Fragments, das an das Substrat gebunden ist, für ein Gen mit höherem Expressionsgrad vermindert ist und für ein Gen mit niedrigerem Expressionsgrad eines Zielgens in Tumorzellen erhöht ist.

6. Das Verfahren gemäß Anspruch 4, worin das Antikrebsmittel vom Antimetabolit-Typ TS-1 ist.

7. Verfahren zur Herstellung eines DNA-Array zur Messung der Sensitivität auf ein Antikrebsmittel vom Antimetabolit-Typ oder auf eine Kombination eines solchen Antikrebsmittels und eines anderen Antikrebsmittels, **dadurch gekennzeichnet, dass** es ein Substrat und mindestens die folgenden Genfragmente (A), (B), (C) und (D), die an das Substrat gebunden sind, umfasst:

(A) ein Thymidylatsynthase Genfragment bestehend aus einer Nukleotidsequenz von SEQ ID NO: 3, ein Dihydropyrimidindehydrogenase Genfragment bestehend aus einer Nukleotidsequenz von SEQ ID NO: 4, ein Orotatphosphoribosyltransferase Genfragment bestehend aus einer Nukleotidsequenz von SEQ ID NO: 5, ein Thymidinphosphorylase Genfragment bestehend aus einer Nukleotidsequenz von SEQ ID NO: 6 und ein Thymidinkinase-1 Genfragment bestehend aus einer Nukleotidsequenz von SEQ ID NO: 7;
(B) ein DNA Excision Repair Protein ERCC1 Genfragment bestehend aus einer Nukleotidsequenz von SEQ ID NO: 28 und ein Uracil-DNA-Glycosylase Genfragment bestehend aus einer Nukleotidsequenz von SEQ ID NO: 29;
(C) ein Topoisomerase 1 Genfragment bestehend aus einer Nukleotidsequenz von SEQ ID NO: 37, ein P-Glycoprotein Genfragment bestehend aus einer Nukleotidsequenz von SEQ ID NO: 38, ein equilibrative nucleoside Transporter 1 Genfragment bestehend aus einer Nukleotidsequenz von SEQ ID NO: 39, und ein multidrug resistence associated Protein-1 Genfragment bestehend aus einer Nukleotidsequenz von SEQ ID NO: 40; und
(D) zwei oder mehr Genfragmente ausgewählt aus einer Haushaltsgengruppe bestehend aus einem Glycerinaldehyd-3-phosphatdehydrogenase Genfragment bestehend aus einer Nukleotidsequenz von SEQ ID NO: 52, einem Beta-Actin Genfragment bestehend aus einer Nukleotidsequenz von SEQ ID NO: 53 und einem 40S ribosomalen Protein S9 Genfragment bestehend aus einer Nukleotidsequenz von SEQ ID NO: 54.

8. Das Verfahren gemäß Anspruch 7, worin das DNA-Array ferner ein proliferating cell nuclear antigen Genfragment bestehend aus einer Nukleotidsequenz von SEQ ID NO: 51 aufweist.

9. Das Verfahren gemäß Anspruch 7, worin die Menge eines betreffenden Fragments, das an das Substrat gebunden ist, für ein Gen mit höherem Expressionsgrad vermindert ist und für ein Gen mit niedrigerem Expressionsgrad eines Zielgens in Tumorzellen erhöht ist.

## Revendications

1. Puce à ADN pour mesurer la sensibilité à un agent anticancéreux de type antimétabolite ou à une combinaison d'un tel agent anticancéreux et d'un autre agent anticancéreux, **caractérisée en ce qu'**elle comprend un substrat et au moins les fragments géniques (A), (B), (C) et (D) suivants rattachés au substrat :

(A) un fragment de gène de thymidylate synthase constitué d'une séquence de nucléotides SEQ ID NO : 3, un fragment de gène de dihydropyrimidine déshydrogénase constitué d'une séquence de nucléotides SEQ ID NO : 4, un fragment de gène d'orotate phosphoribosyltransférase constitué d'une séquence de nucléotides SEQ ID NO : 5, un fragment de gène de thymidine phosphorylase constitué d'une séquence de nucléotides de la SEQ ID NO : 6, et un fragment de gène de thymidine kinase 1 constitué d'une séquence de nucléotides SEQ ID NO : 7 ;
(B) un fragment du gène ERCC1 de protéine de réparation d'excision d'ADN, constitué d'une séquence de nucléotides SEQ ID NO : 28 et un fragment de gène d'uracile-ADN glycosylase constitué d'une séquence de nucléotides SEQ ID NO : 29 ;
(C) un fragment de gène de topoisomérase 1 constitué d'une séquence de nucléotides de la SEQ ID NO : 37, un fragment de gène de glycoprotéine P constitué d'une séquence de nucléotides SEQ ID NO : 38, un fragment de gène de transporteur de nucléoside d'équilibrage 1, constitué d'une séquence de nucléotides SEQ ID NO : 39, et un fragment de gène de protéine 1 associée à une multirésistance aux médicaments constitué d'une séquence de nucléotides SEQ ID NO : 40 ; et
(D) deux ou plus de deux fragments géniques choisis dans un groupe de gènes domestiques constitué par un fragment de gène de glycéraldéhyde-3-phosphate déshydrogénase constitué d'une séquence de nucléotides SEQ ID NO : 52, un fragment de gène de β-actine constitué d'une séquence de nucléotides SEQ ID NO : 53, et un fragment de gène de la protéine S9 du ribosomiale 40S, constitué d'une séquence de nucléotides SEQ ID NO : 54.

**2.** Puce à ADN selon la revendication 1, dans laquelle chacun des fragments géniques a une taille de 200 à 600 pb.

**3.** Puce à ADN selon la revendication 1, laquelle puce à ADN comprend en outre un fragment de gène d'antigène nucléaire de cellules de prolifération, constitué d'une séquence de nucléotides SEQ ID NO : 51 rattaché audit substrat.

**4.** Procédé pour mesurer la sensibilité d'un échantillon de fluide corporel ou d'un échantillon de tissu d'un patient cancéreux à un agent anticancéreux de type antimétabolite ou à une combinaison d'un tel agent anticancéreux et d'un autre agent anticancéreux, **caractérisé en ce qu'**il comprend l'hybridation d'une puce à ADN, telle que définie dans la revendication 1, 2 ou 3, avec des sondes d'ADNc marquées synthétisées par utilisation, en tant que matrice, d'ARNm obtenu à partir de l'échantillon.

**5.** Procédé selon la revendication 4, dans lequel la quantité d'un fragment respectif rattaché au substrat est réduite pour un gène ayant un niveau d'expression plus élevé et augmentée pour un gène ayant un niveau d'expression plus faible d'un gène cible dans des cellules tumorales.

**6.** Procédé selon la revendication 4, dans lequel ledit agent anticancéreux de type antimétabolite est TS-1.

**7.** Procédé de production d'une puce à ADN pour mesurer la sensibilité à un agent anticancéreux de type antimétabolite ou à une combinaison d'un tel agent anticancéreux et d'un autre agent anticancéreux, **caractérisée en ce qu'**elle comprend un substrat et au moins les fragments géniques (A), (B), (C) et (D) suivants rattachés au substrat :

(A) un fragment de gène de thymidylate synthase constitué d'une séquence de nucléotides SEQ ID NO : 3, un fragment de gène de dihydropyrimidine déshydrogénase constitué d'une séquence de nucléotides SEQ ID NO : 4, un fragment de gène d'orotate phosphoribosyltransférase constitué d'une séquence de nucléotides SEQ ID NO : 5, un fragment de gène de thymidine phosphorylase constitué d'une séquence de nucléotides SEQ ID NO : 6, et un fragment de gène de thymidine kinase 1 constitué d'une séquence de nucléotides SEQ ID NO : 7 ;
(B) un fragment du gène ERCC1de protéine de réparation d'excision d'ADN, constitué d'une séquence de nucléotides SEQ ID NO : 28 et un fragment de gène d'uracile-ADN glycosylase constitué d'une séquence de nucléotides SEQ ID NO : 29 ;
(C) un fragment de gène de topoisomérase 1 constitué d'une séquence de nucléotides SEQ ID NO : 37, un fragment de gène de glycoprotéine P constitué d'une séquence de nucléotides de la SEQ ID NO : 38, un fragment de gène de transporteur de nucléoside d'équilibrage 1, constitué d'une séquence de nucléotides SEQ ID NO : 39, et un fragment de gène de protéine 1 associée à une multirésistance aux médicaments constitué d'une séquence de nucléotides SEQ ID NO : 40 ; et
(D) deux ou plus de deux fragments géniques choisis dans un groupe de gènes domestiques constitué par un fragment de gène de glycéraldéhyde-3-phosphate déshydrogénase constitué d'une séquence de nucléotides SEQ ID NO : 52, un fragment de gène de β-actine constitué d'une séquence de nucléotides SEQ ID NO : 53, et un fragment de gène de la protéine S9 du ribosome 40S, constitué d'une séquence de nucléotides SEQ ID NO : 54.

**8.** Procédé selon la revendication 7, dans lequel ladite puce à ADN comprend en outre un fragment de gène d'antigène nucléaire de cellules de prolifération, constitué d'une séquence de nucléotides SEQ ID NO : 51.

**9.** Procédé selon la revendication 7, dans lequel la quantité d'un fragment respectif rattaché au substrat est réduite pour un gène ayant un niveau d'expression plus élevé et augmentée pour un gène ayant un niveau d'expression plus faible d'un gène cible dans des cellules tumorales.

Fig. 1

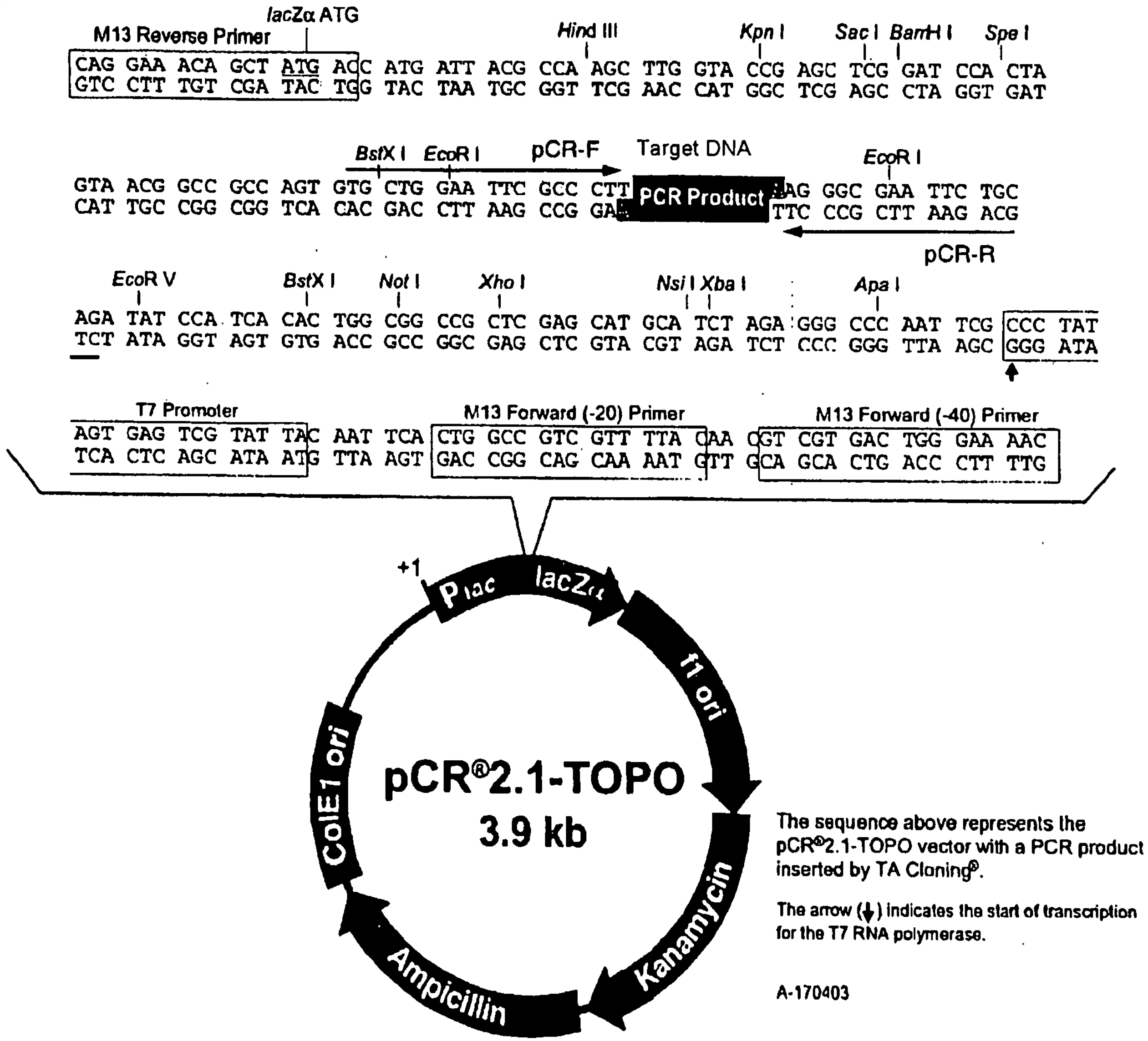


The sequence above represents the pCR®2.1-TOPO vector with a PCR product inserted by TA Cloning®.

The arrow (↓) indicates the start of transcription for the T7 RNA polymerase.

A-170403

Fig. 2

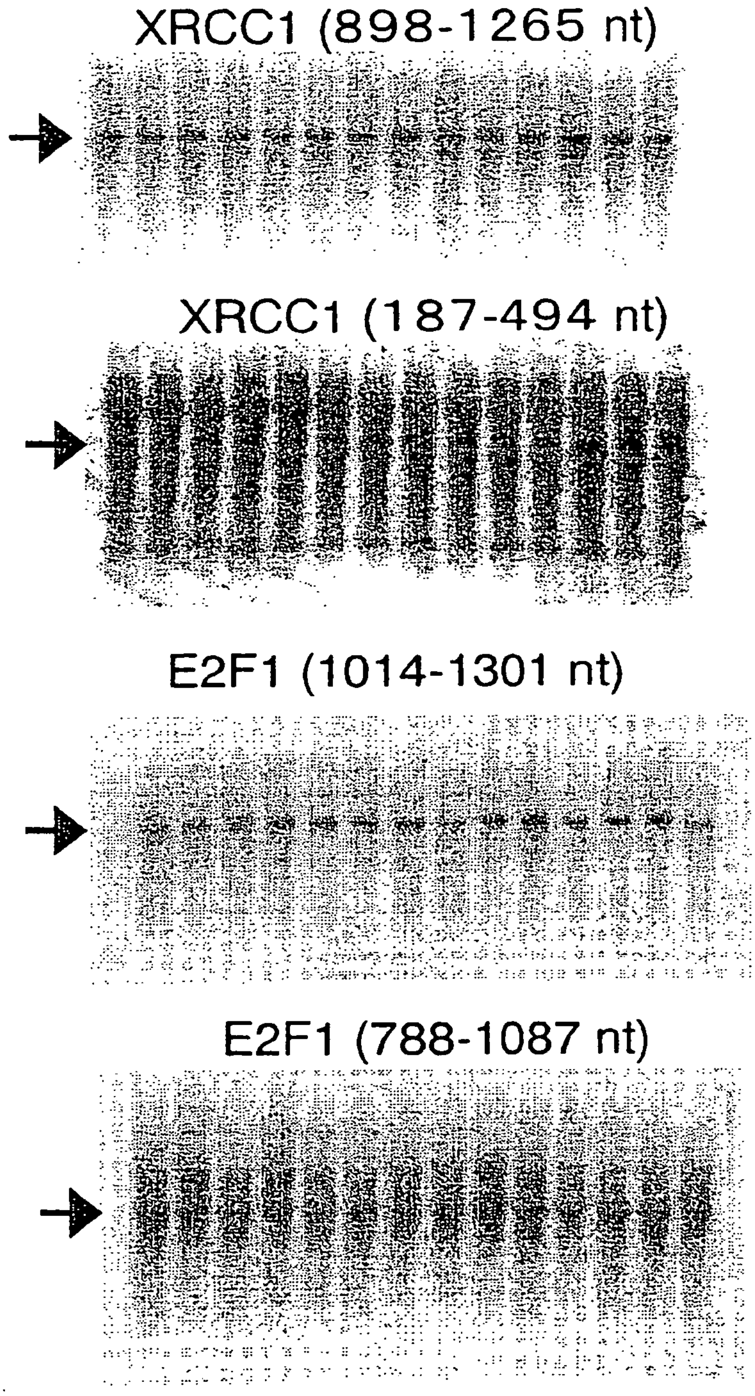
XRCC1 (898-1265 nt)
XRCC1 (187-494 nt)
E2F1 (1014-1301 nt)
E2F1 (788-1087 nt)

Fig. 3

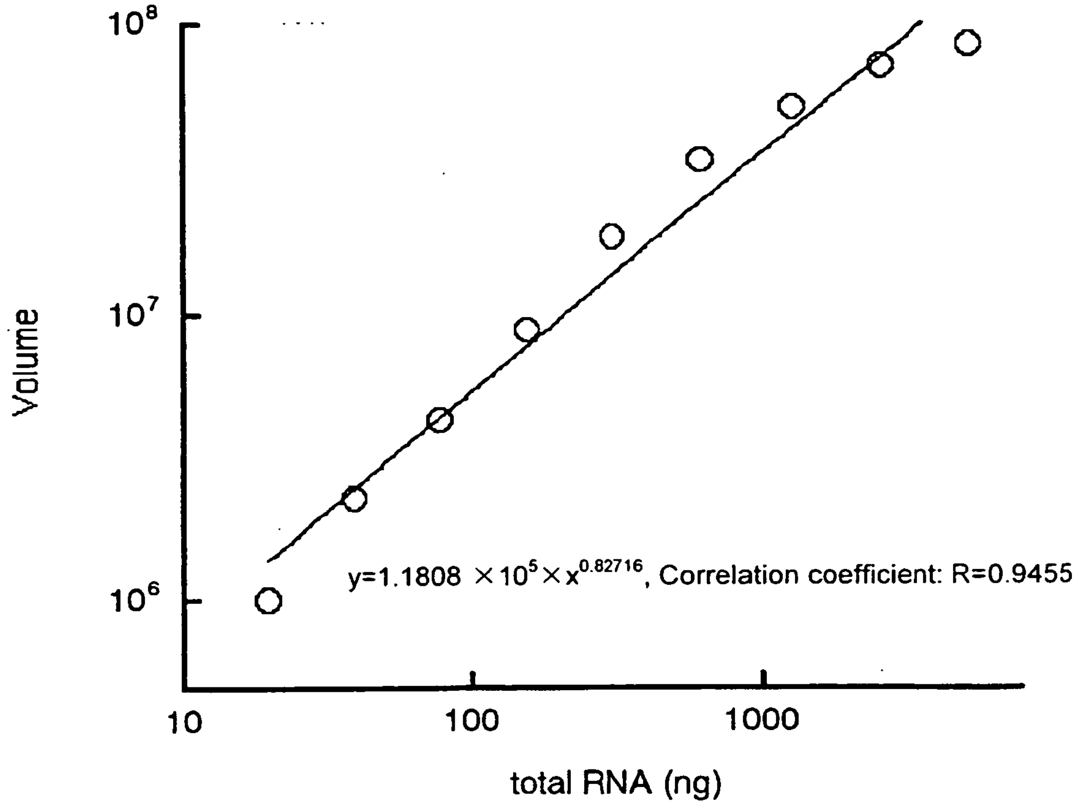
10^8
10^7
10^6
Volume
10
100
1000
total RNA (ng)
y=1.1808 ×10^5×x^0.82716, Correlation coefficient: R=0.9455

Fig. 4

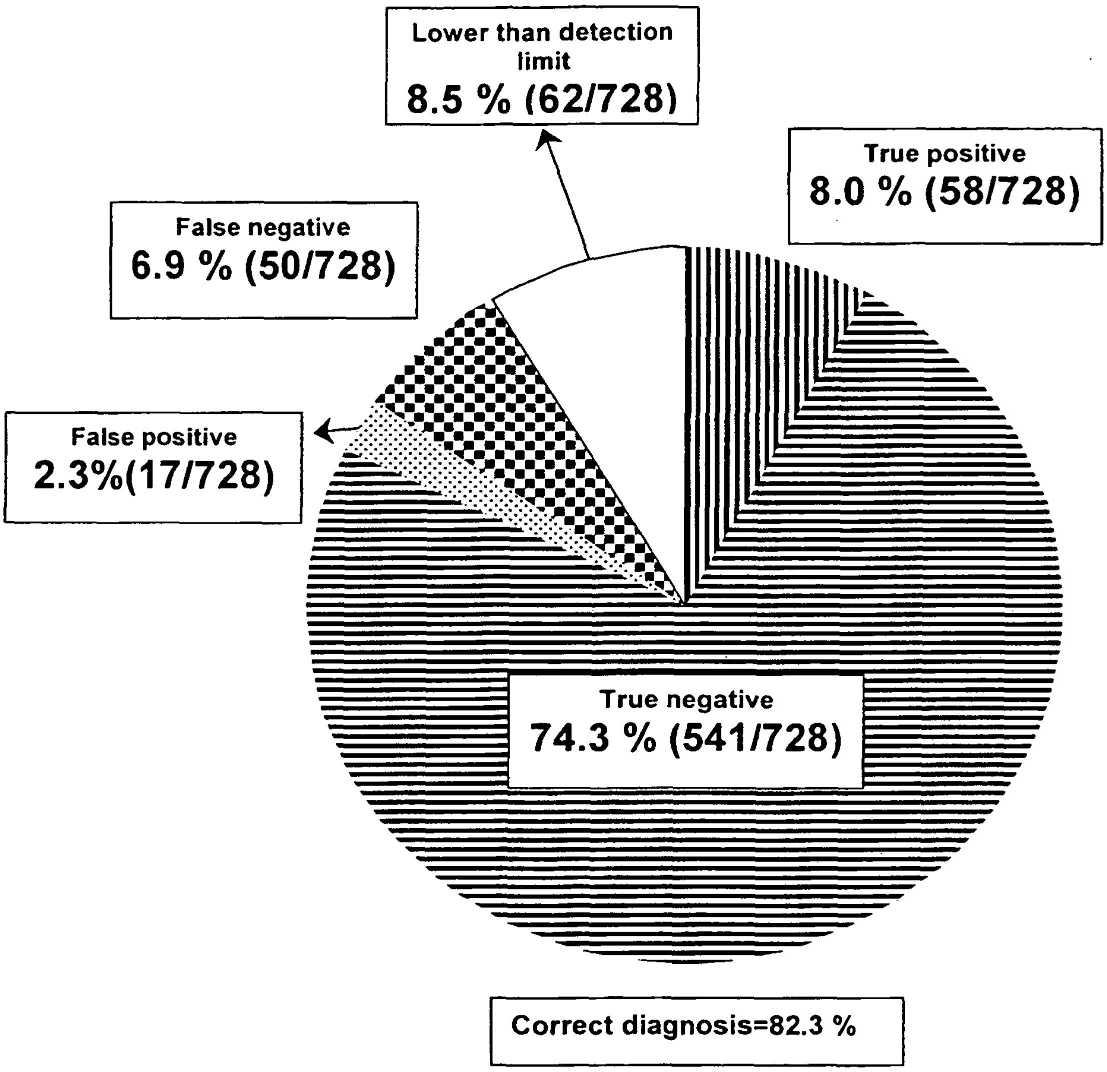
Lower than detection limit
8.5 % (62/728)
True positive
8.0 % (58/728)
False negative
6.9 % (50/728)
False positive
2.3%(17/728)
True negative
74.3 % (541/728)
Correct diagnosis=82.3 %

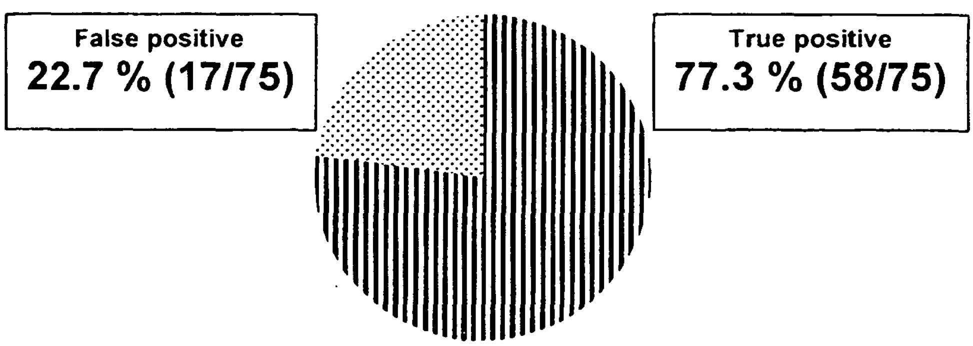
False positive
22.7 % (17/75)
True positive
77.3 % (58/75)

Fig. 5

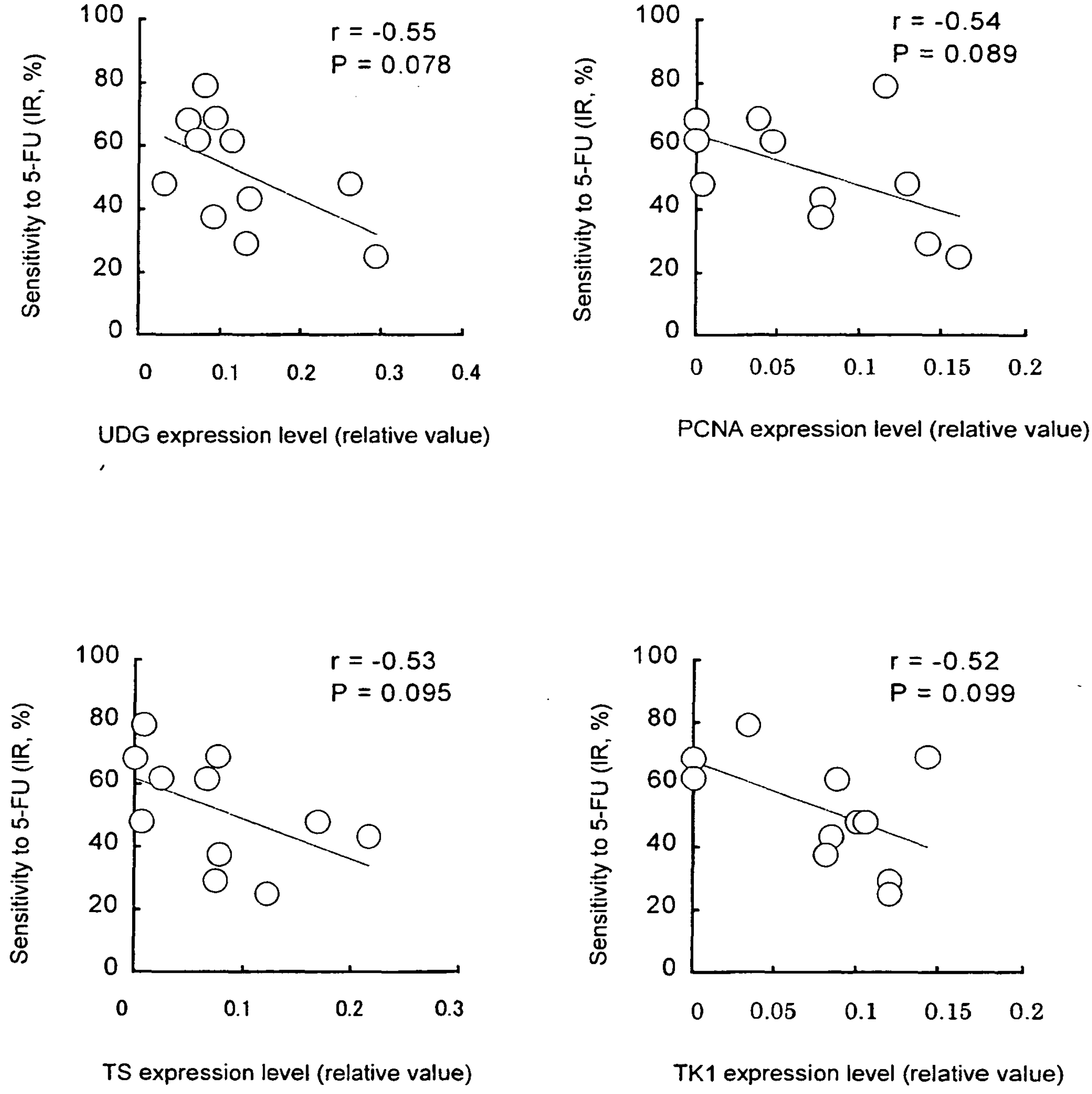
r = -0.55
P = 0.078
Sensitivity to 5-FU (IR, %)
100
80
60
40
20
0
0
0.1
0.2
0.3
0.4
UDG expression level (relative value)
r = -0.54
P = 0.089
Sensitivity to 5-FU (IR, %)
0
0.05
0.1
0.15
0.2
PCNA expression level (relative value)
r = -0.53
P = 0.095
Sensitivity to 5-FU (IR, %)
TS expression level (relative value)
r = -0.52
P = 0.099
Sensitivity to 5-FU (IR, %)
TK1 expression level (relative value)

Fig 6

Measurement of TS expression level by use of the DNA array of the present invention

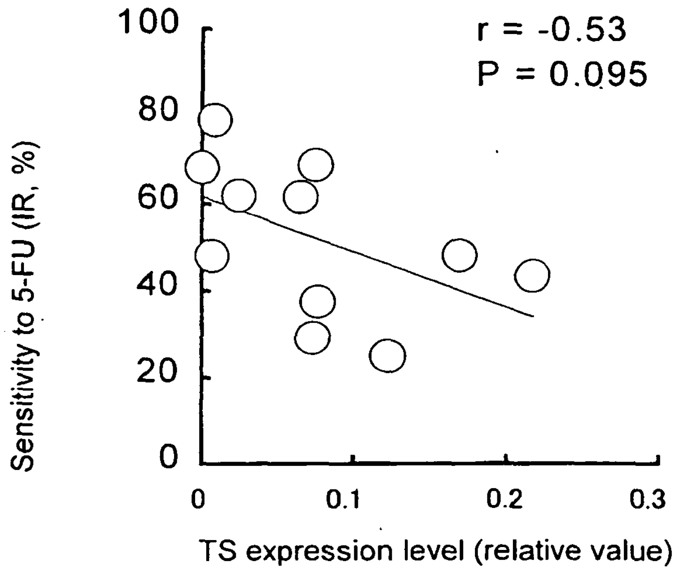


Measurement of TS expression level through real time RT-PCR

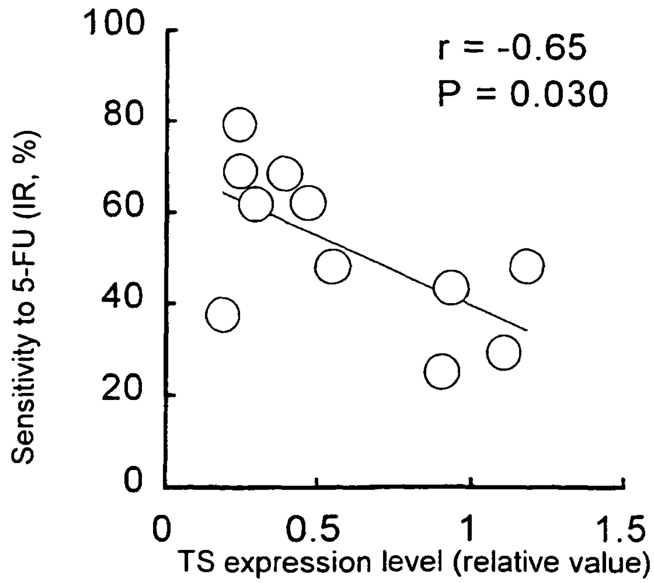

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0132928 A
- JP 10503841 W
- JP P2001204448 B
- JP P2001239181 B


### Non-patent literature cited in the description


- *Clinical Cancer Research,* 1999, vol. 5, 883-889
- *Cancer and Chemotherapy,* 1997, vol. 24 (6), 705-712
- *Clinical Cancer Research,* 2000, vol. 6, 1322-1327
- *Journal of Clinical Oncology,* 1998, vol. 16, 309-316